(19) Europäisches Patentamt
European Patent Office
Office européen des brevets

(11) **EP 2 780 476 B1**

(12) **EUROPEAN PATENT SPECIFICATION**

(45) Date of publication and mention
of the grant of the patent:
**09.01.2019 Bulletin 2019/02**

(51) Int Cl.:
***C12Q 1/6886*** (2018.01)

(21) Application number: **12849353.3**

(22) Date of filing: **19.11.2012**

(86) International application number:
**PCT/SG2012/000435**

(87) International publication number:
**WO 2013/074044 (23.05.2013 Gazette 2013/21)**

(54) **METHODS FOR DIAGNOSIS AND/OR PROGNOSIS OF GYNECOLOGICAL CANCER**

VERFAHREN ZUR DIAGNOSE UND/ODER PROGNOSE VON GYNÄKOLOGISCHEN KARZINOMEN

MÉTHODES DE DIAGNOSTIC ET/OU DE PRONOSTIC D'UN CANCER GYNÉCOLOGIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.11.2011  SG 201108632**

(43) Date of publication of application:
**24.09.2014  Bulletin 2014/39**

(73) Proprietor: **Agency For Science, Technology And Research (A*star)**
**Singapore 138632 (SG)**

(72) Inventors:
- **BATAGOV, Arsen**
  **Singapore 138671 (SG)**
- **IVSHINA, Anna**
  **Singapore 138671 (SG)**
- **KUZNETSOV, Vladimir**
  **Singapore 138671 (SG)**

(74) Representative: **Bailey, Jennifer Ann et al**
**Marks & Clerk LLP**
**Alpha Tower**
**Suffolk Street Queensway**
**Birmingham B1 1TT (GB)**

(56) References cited:
| | |
|---|---|
| WO-A1-2011/146938 | WO-A2-01/18542 |
| WO-A2-02/071928 | WO-A2-02/079411 |
| WO-A2-2007/099396 | CN-A- 101 386 888 |
| US-A- 5 872 104 | US-A1- 2005 244 851 |
| US-A1- 2009 062 131 | |

- **FERRIS: "POSTER SESSION", GYNECOLOGIC ONCOLOGY, vol. 108, no. 3, 1 March 2008 (2008-03-01) , page S133, XP055200827, ISSN: 0090-8258, DOI: 10.1016/j.ygyno.2007.11.037**
- **BROOKS D J ET AL: "EXPRESSION OF THE ZINE FINGER GENE EVI-1 IN OVARIAN AND OTHER CANCERS", BRITISH JOURNAL OF CANCER, NATURE PUBLISHING GROUP, GB, vol. 74, no. 10, 1 January 1996 (1996-01-01), pages 1518-1525, XP008027058, ISSN: 0007-0920**
- **Michael Johnson ET AL: "Identification of EVI1 amplification in ovarian cancer by oligonucleotide array comparative genomic hybridization", Cancer Research, 1 January 2007 (2007-01-01), XP055200812, Retrieved from the Internet: URL:http://cancerres.aacrjournals.org/cont ent/67/9_Supplement/1172.short [retrieved on 2015-07-08]**
- **DATABASE MEDLINE [Online] US NATIONAL LIBRARY OF MEDICINE (NLM), BETHESDA, MD, US; July 1990 (1990-07), MORISHITA K ET AL: "Unique expression of the human Evi-1 gene in an endometrial carcinoma cell line: sequence of cDNAs and structure of alternatively spliced transcripts.", XP002741897, Database accession no. NLM2115646 & ONCOGENE JUL 1990, vol. 5, no. 7, July 1990 (1990-07), pages 963-971, ISSN: 0950-9232**
- **NANJUNDAN, M. ET AL.: 'Amplification of MDS1/EVI1 and EVI1, located in the 3q26.2 amplicon, is associated with favorable patient prognosis in ovarian cancer' CANCER RESEARCH vol. 67, 2007, pages 3074 - 3084, XP055154866**

**(Cont. next page)**

EP 2 780 476 B1

- JAZAERI, A. A. ET AL.: ''Evaluation of EVI1 and EVIIs (Delta324) as potential therapeutic targets in ovarian cancer' GYNECOLOGIC ONCOLOGY vol. 118, 2010, pages 189 - 95, XP027117453
- QUINN, M. C. J. ET AL.: 'Reprogramming of the transcriptome in a novel chromosome 3 I transfer tumor suppressor ovarian cancer cell line model affected molecular networks I that are characteristic of ovarian cancer' MOLECULAR CARCINOGENESIS vol. 48, 2009, pages 648 - 661, XP055154870
- SUNDE, J. S ET AL.: 'Expression profiling identifies altered expression of genes that contribute to the inhibition of transforming growth factor-beta signaling in ovarian cancer' CANCER RESEARCH vol. 66, 2006, pages 8404 - 8412, XP002511191
- DUTTA PUNASHI ET AL: "EVI1 splice variants modulate functional responses in ovarian cancer cells", MOLECULAR ONCOLOGY, ELSEVIER, AMSTERDAM, NL, vol. 7, no. 3, 5 March 2013 (2013-03-05), pages 647-668, XP028543815, ISSN: 1574-7891, DOI: 10.1016/J.MOLONC.2013.02.008

**Description**

**FIELD OF THE INVENTION**

The present invention relates to method(s) for diagnosis and/or prognosis of cancer in a subject and in particular but not exclusively by analyzing MDS1 and EVI1 complex locus (also known as MECOM locus).

**BACKGROUND TO THE INVENTION**

Cancers are one of the most leading causes of cancers in the world. Ovarian cancer and cervical cancer amongst others are very common cancers that affect women worldwide. Out of all the cancers that affect women, epithelial ovarian cancer (EOC) ranks fifth when considering woman cancer mortality in the world and fourth for the age group 40 to 59 years old, with overall a 5-year survival rate of women inflicted by EOC of only 46%. This is despite improvements in surgical techniques and the advent of more targeted therapeutics such as bevacizimab. The low survival rate is explained by the lack of diagnosis of EOC at an early stage, acquired resistance to chemotherapy in a significant number of patients over the years and the lack of effective therapies for advanced refractory disease. The 5-year survival rate is up to 95% if EOC diagnosis is made in the early stage of the disease.

EOC comprises three major histological subtypes; serous, mucinous and endometrioid. Serous EOC includes serous cystomas, serous benign cystadenomas, serous cystadenomas with proliferating activity of the epithelial cells and nuclear abnormalities but with no infiltrative destructive growth (low potential or borderline malignancy), and serous cystadenocarcinomas. Mucinous EOC includes mucinous cystomas, mucinous benign cystadenomas, mucinous cystadenomas with proliferating activity of the epithelial cells and nuclear abnormalities but with no infiltrative destructive growth (low potential or borderline malignancy), and mucinous cystadenocarcinomas. Endometrioid EOC includes endometrioid tumors (similar to adenocarcinomas in the endometrium), endometrioid benign cysts, endometrioid tumors with proliferating activity of the epithelial cells and nuclear abnormalities but with no infiltrative destructive growth (low malignant potential or borderline malignancy), and endometrioid adenocarcinomas. Two further, less-prevalent histological subtypes also exist, clear cell and undifferentiated.

EOC may also be categorized by "stages", depending upon how far they have spread beyond the ovary. Thus, Stage I is defined as ovarian cancer that is confined to one or both ovaries. Stage II is defined as ovarian cancer that has spread to pelvic organs (e.g., uterus, fallopian tubes), but has not spread to abdominal organs. Stage III is defined as ovarian cancer that has spread to abdominal organs or the lymphatic system (e.g., pelvic or abdominal lymph nodes, on the liver, on the bowel). Finally, Stage IV is defined as ovarian cancer that has spread to distant sites (e.g., lung, inside the liver, brain, lymph nodes in the neck).

EOCs may be graded according to the appearance of the cancer cells. Low-grade (or Grade 1) means that the cancer cells look very like the normal cells of the ovary; they usually grow slowly and are less likely to spread. Moderate-grade (or Grade 2) means that the cells look more abnormal than low-grade cells. High-grade (or Grade 3) means that the cells look very abnormal. They are likely to grow more quickly and are more likely to spread.

EOC, like most other cancers, is thus a complex heterogeneous disease, influenced and controlled by multiple genetic and epigenetic alterations leading to an increasingly aggressive phenotype. It is now well recognised that the characteristics of an individual tumor and its life course results from multiple somatic mutations acquired over time (e.g. TP53, PTEN, RAS) and continual evolution of the host responses to environmental factors. From a therapeutic standpoint EOC is best considered a collection of complex inter-related diseases represented by an immense natural heterogeneity in tumor phenotypes, disease outcomes, and response to treatment.

A major challenge is thus to identify and thoroughly validate diagnostic and prognostic biomarkers that can accurately describe the heterogeneity ascribed to EOC. In addition, accurate predictive biomarkers are required to guide current treatment protocols, as well as to guide the development and application of new targeted therapies. CA-125 (MUC16, Cancer antigen 125) protein is currently considered the best diagnostic marker of EOC. However, the true positive rate of MUC16 test is only about 50% of stage I EOC patients, while it returns more than 80% of true positives for patients at stages I-IV. About 25% of EOCs especially at the early stages do not produce reliably-detectable CA-125 and therefore its application in clinical settings is limited. It has been reported that CA-125 in combination with human epididymis protein 4 (HE4) is elevated in greater than 90% of EOC. Such poor prognostic statistics indicate that there is an urgent need to improve understanding of the molecular mechanisms underlying EOC, so as to develop better prognostic and predictive assays and identify new therapeutic targets.

Currently there are about 15 oncogenes considered particularly important for ovarian cancers and 11 of them, including EVI1 have been shown to be amplified in the genome of cancer cells. Proto-oncogene EVI1 (ectopic viral integration site 1), encoded by the MECOM locus (Entrez GeneID: 2122) located at the 3q26 chromosome region, is amplified in many cancers. EVI1 protein was identified as an evolutionary conserved transcription factor sharing 94% amino acid sequence homology between human and mice. In the adult human tissues it is highly expressed in kidney, lung, pancreas, brain and ovaries. In mouse embryos it is highly expressed in the urinary system, lungs and heart and its activity is vital for the embryonic development. The majority of research of EVI1 describes its significance in pathology. If overexpressed in blood cells, EVI1 has been shown to produce a number of alternatively spliced transcripts and causes various hematopoietic disorders, including myeloid leukemias. EVI1 was found to be overexpressed in the blood of up to 21% patients with acute myeloid leukemia (AML). In 4% of AML cases chromosome region 3q is aberrated. High expression of EVI1, regardless the amplification of MECOM locus alone was recently found to be a significant survival factor for ovarian cancer patients. Chromosome region 3q25-27 is amplified in cancers in a various organs: ovary, cervix, lung, oesophagus, colon, head and neck and prostate. Amplification of MECOM is also associated with resistance to chemotherapy in EOC.

However, the information about the role of MECOM locus in ovarian cancer development has been contradictory. In a recent research, although remarkably high expression of EVI1 was detected in serous ovarian cancer tissues, fallopian tube fimbria and benign neoplasms, in comparison with normal tissues of different types, no influence of EVI1 transcript expression on cancer cell proliferation, induced apoptosis or double-strand DNA breaks was revealed in the tests of OVCAR8 cell culture (Jazaeri AA, 2010). In another research it was reported that amplification of MECOM locus can be associated with favorable patient prognosis in ovarian cancer (Nanjundan et al, 2007). Ferris et al., 2008 discloses that EVI1 is expressed in some epithelial ovarian cancers. Brooks et al., 1996 is concerned with the expression of EVI1 in endometrial and other cancers, while Johnson et al., 2007 discloses that EVI1 is overexpressed and has DNA copy number alterations in serous ovarian tumour samples compared to normal female lymphocytes. Morishita K et al., 1990 states that there is low expression of EVI1 in one human endometrial carcinoma cell line. A kit for detecting in situ hybridization of an EVI1 gene is disclosed in CN101386888. WO01/18542 considers the sequence of EVI1. A micro-array useful as a prognostic tool for CC-RCC is disclosed in WO02/079411, and Nanjundan et al., 2007 is concerned with the prognosis of ovarian cancer.

Accordingly, there is still a need in determining if EVI1 can be used as a biomarker in diagnosis of EOC and to find improved methods for the diagnosis and prognosis of EOC.

## SUMMARY OF THE INVENTION

The markers currently being used for detection of EOC lack adequate sensitivity and specificity to be applicable in large populations particularly during the early stages of ovarian cancer. The present invention attempts to fill this gap by proposing MECOM locus and its genes including non-limiting examples, EVI1 and/or MDS1 as more sensitive and specific diagnostic markers than MUC16, as well as other widely accepted biomarkers. In particular, the present invention relates to identification of clinically distinct subgroups of EOC patients differentially characterized in one of the following aspects:

- ovarian tumor primary origin (primary EOC versus secondary metastasis),
- presence of ovarian cancer,
- tumor malignancy potential,
- patient's survival prognosis, and
- effectiveness of treatment.

The identification of patient groups is achieved in the integrative analysis of DNA copy number variations and/or gene expression level of at least one gene from the MDS1 and/or EVI1 complex locus (also known as MECOM locus) in the tumor samples. Cutoff values for DNA copy number values of MECOM locus and expression levels of EVI1 and MDS1 genes belonging to this locus maximally separating the patient groups by the chosen criteria are obtained separately for MECOM locus genes: EVI1 and MDS1.

In particular, the diagnostic procedure for individual patients may include the steps of:

- obtaining tumor sample material;
- measurement of DNA copy number value of EVI1 and/or MDS1 gene;
- measurement of gene expression level (using a non-limiting example of mRNA) of EVI1 and/or MDS1 genes;
- comparison of the values obtained in the measurements of the above steps against the DNA copy number and/or expression cutoff values respectively.

According to one aspect, the present invention provides an *in vitro* method for obtaining information in relation to a medical condition in a subject, the method comprising determining in a sample of the subject:

(i) gene expression level of at least one gene in the MECOM locus, selected from *EVI1* and *MOS1*; and/or
(ii) copy number of at least one gene in the MECOM locus selected from *EVI1* and *MOS1;*

wherein the information is selected from the group consisting of:

a) whether the subject has epithelial ovarian cancer, preferably high grade serous ovarian carcinoma and/or a predisposition to epithelial ovarian cancer, preferably high grade serous ovarian carcinoma, wherein the gene expression level against at least one statistically optimized gene expression cutoff value derived from a plurality of training subjects with known diagnosis and/or the at least one gene copy number against at least one statistically optimized gene copy number cutoff value derived from a plurality of training subjects with known diagnosis is/are indicative of said information, wherein said optimized cutoff value is obtained with the following steps;

(i) extract the expression level or copy number level of a gene for each training subject;
(ii) obtain the cumulative distribution function of the gene expression or copy number levels of two sets of training subjects, one set being associated with a positive diagnosis and one set being associated with a negative diagnosis; and
(iii) select a cut-off value which divides the training subjects into two groups, based on whether the gene expression level or copy number level of each training subject is higher or lower than the cut-off value, and which minimizes the false positive and/or false negative rate in the diagnosis using one-dimensional statistical optimization;
or, if the expression and the copy number values are identified in the same sample, the optimal cutoff for both expression and copy number are obtained simultaneously by minimising the false positive and/or false negative rates using two-dimensional statistical optimization;

wherein said epithelial ovarian cancer determination comprises using PCR primers comprising a forward primer SEQ ID NO: 1 and reverse primer SEQ ID NO: 2 and probe SEQ ID NO: 3, or using at least one of the probe sets selected from 1881_g_at, 1882_g_at, 208434_at, 221884_at and 226420_at;
b) survival prognosis of the subject when the subject has epithelial ovarian cancer, preferably high grade serous ovarian carcinoma, wherein the expression level against at least one statistically optimized gene expression cutoff value and/or the at least one gene copy number against at least one statistically optimized gene copy number cutoff value is/are indicative of said information, wherein, for primary patient stratification, the optimal copy number and expression cutoff values are obtained for each gene using a two-step optimization algorithm as follows;

(i) extract the expression level and copy number of the gene for each training subject;
(ii) statistically estimate a copy number cutoff value and divide the training subjects into two cohorts according to whether the copy number of the gene is above or below the estimated copy number cutoff value; and
(iii) for each said cohort, select a gene expression cutoff value that divides the training subjects in each cohort into two groups based on whether the gene expression level of each training subject is higher or lower than the expression cutoff value, wherein the cutoff value minimizes the Wald test P-value of Kaplan-Meier survival models;

wherein said survival prediction determination comprises using PCR primers comprising a forward primer SEQ ID NO: 1 and reverse primer SEQ ID NO: 2 and probe SEQ ID NO: 3, or using at least one of the probe sets selected from 1881_g_at, 1882_g_at, 221884_at and 226420_at;
c) effectiveness of treatment of epithelial ovarian cancer, preferably high grade serous ovarian carcinoma, in the subject, wherein the gene expression level against at least one statistically optimized gene expression cutoff value and/or the at least one gene copy number against at least one optimized gene copy number cutoff value are indicative of said information;
wherein said effectiveness of treatment of epithelial ovarian cancer determination comprises using PCR primers comprising a forward primer SEQ ID NO: 1 and reverse primer SEQ ID NO: 2 and probe SEQ ID NO: 3, or using at least one of the probe sets selected from 1881_g_at, 1882_g_at, 221884_at and 226420_at;
d) whether epithelial ovarian cancer, preferably high grade serous ovarian carcinoma, in the subject is of primary or secondary origin, wherein the gene expression level against at least one statistically optimized gene expression cutoff value and/or the at least one gene copy number against at least one statistically optimized gene copy number cutoff value are indicative of said information;
wherein said determination of whether the epithelial ovarian cancer is of primary or secondary origin comprises using PCR primers comprising a forward primer SEQ ID NO: 1 and reverse primer SEQ ID NO: 2 and probe SEQ ID NO: 3, or using at least one of the probe sets selected from 226420_at, 221884_at and 208434_at; and
e) whether the subject has high grade serous ovarian carcinoma, cervical cancer, ovarian endometriosis, clear cell renal carcinoma and/or a predisposition to cervical cancer, ovarian endometriosis, clear cell renal carcinoma, wherein the level against at least one statistically optimized gene expression cutoff value and/or the at least one gene copy number against at least one statistically optimized gene copy number cutoff value are indicative of the subject having high grade serous ovarian carcinoma, cervical cancer, endometriosis, clear cell renal carcinoma and/or a predisposition to cervical cancer, endometriosis, or clear cell renal carcinoma;

wherein said determination of the type of cancer comprises using PCR primers comprising a forward primer SEQ ID NO: 1 and reverse primer SEQ ID NO: 2 and probe SEQ ID NO: 3, or using at least one of the probe sets selected from 226420_at, 1881_g_at, 1882_g_at, 221884_at and 208434_at,

wherein said gene expression level in a)-e) is determined from at least one transcript variant or isoform comprising a nucleic acid sequence selected from the group consisting of SEQ ID NO: 4, SEQ ID NO: 5, SEQ ID NO: 11, SEQ ID NO: 12, SEQ ID NO: 13, SEQ ID NO: 14 and SEQ ID NO: 15.

Embodiments are provided by the dependent claims.

According to a second aspect, there is provided the use of specific oligonucleotides derived from one or more of the individual probe sets selected from the group consisting of 1881_g_at, 1882_g_at, 208434_at; 221884_at and 226420_at, their fragments, derivatives, mutants and complementary sequences thereof, in a method according to the first aspect.

According to one related aspect, the present invention relates to an *in vitro* method for diagnosing EOC and/or predisposition to epithelial ovarian cancer in a subject and/or determining survival prognosis of a subject with epithelial ovarian cancer, and/or determining effectiveness of treatment of epithelial ovarian cancer, and/or determining if an epithelial ovarian cancer in a subject is of primary origin or secondary origin, the method comprising determining in a sample of the subject:

(i) gene expression level (using a non-limiting example of mRNA) of at least one gene in MECOM locus; and/or
(ii) DNA copy number value of at least one gene in MECOM locus;

wherein the level against at least one expression cutoff value and/or DNA copy number against at least one DNA copy number cutoff value are indicative of the subject having EOC, or predisposition to EOC, or the survival prognosis of the subject or the effectiveness of the treatment on the subject.

According to a further related aspect, there is provided a nucleotide sequence selected from the group consisting of SEQ ID NOs: 1-3, fragments, derivatives, variants and complementary sequences thereof

The present invention also provides the use of a kit comprising a forward amplification primer having a sequence comprising SEQ ID NO: 1, a reverse amplification primer having a sequence comprising SEQ ID NO: 2 and a probe having a sequence comprising SEQ ID NO: 3 or specific oligonucleotides derived from one or more of the individual probe sets selected from the group comprising 1881_g_at, 1882_g_at, 208434_at; 221884_at and 226420_at, their fragments, derivatives, mutants and complementary sequences thereof in a method for obtaining information in relation to a medical condition in a subject according to the method of the first aspect.

Also provided is a set of oligonucleotides consisting of,

a forward amplification primer having a nucleic acid sequence comprising SEQ ID NO: 1, a reverse amplification primer having a nucleic acid sequence comprising SEQ ID NO: 2 and a probe having a nucleic acid sequence comprising SEQ ID NO: 3,

wherein the set is capable of simultaneous detection and discrimination of complete protein-coding specific isoforms of EVI1 isoform only, MDS1 only and the combination of EVI1 and EVI1/MDS1 isoforms as defined by SEQ ID NO: 4, SEQ ID NO: 5, SEQ ID NO: 11, SEQ ID NO: 12, SEQ ID NO: 13, SEQ ID NO: 14 and SEQ ID NO: 15, and complete determination of transcription status of whole MECOM locus in an individual patient sample.

Also described are kits, computer programs, and computer systems using the method according to any aspect of the present invention.

## BRIEF DESCRIPTION OF DRAWINGS

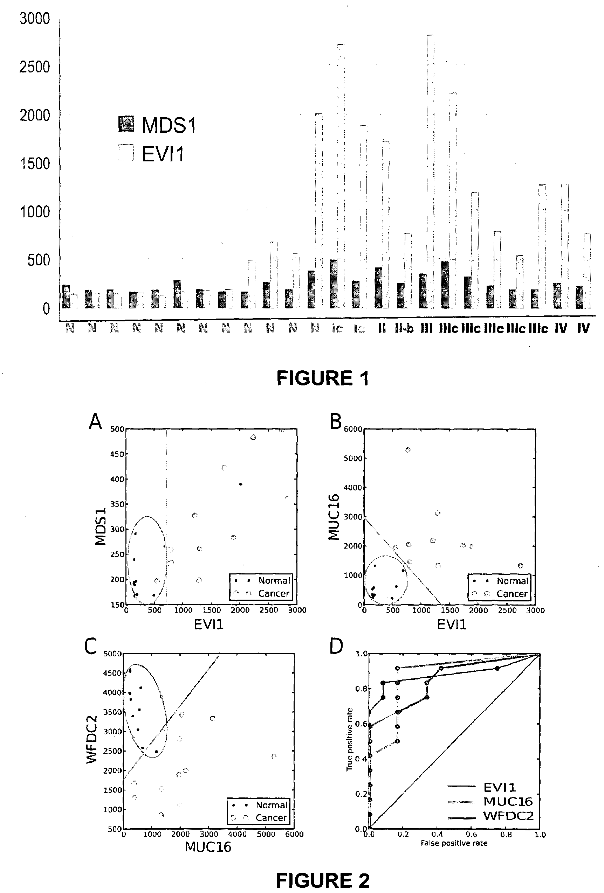

Figure 1 is a graph showing that the expression of EVI1 gene of MECOM locus gene was strongly increased in ovarian cancer cells, in comparison with normal epithelium. Absciss axis - EOC tissue samples of individual patients at EOC stages I-IV and normal ovarian tissue from patients with non-cancerous gynecological diseases. Ordinate axis - MDS1 and EVI1 expression microarray signal.

Figure 2 are four graphs (A-D) with A and B showing that MECOM locus genes expression microarray signals in combination with each other and with MUC16 discriminates between normal and cancerous ovarian epithelia better than the current best clinically used biomarker combination MUC16-WFDC2 (C). D is a comparison of ROC curves of MECOM locus gene EVI1 with the current best clinical diagnostic markers WFDC2 and MUC16.

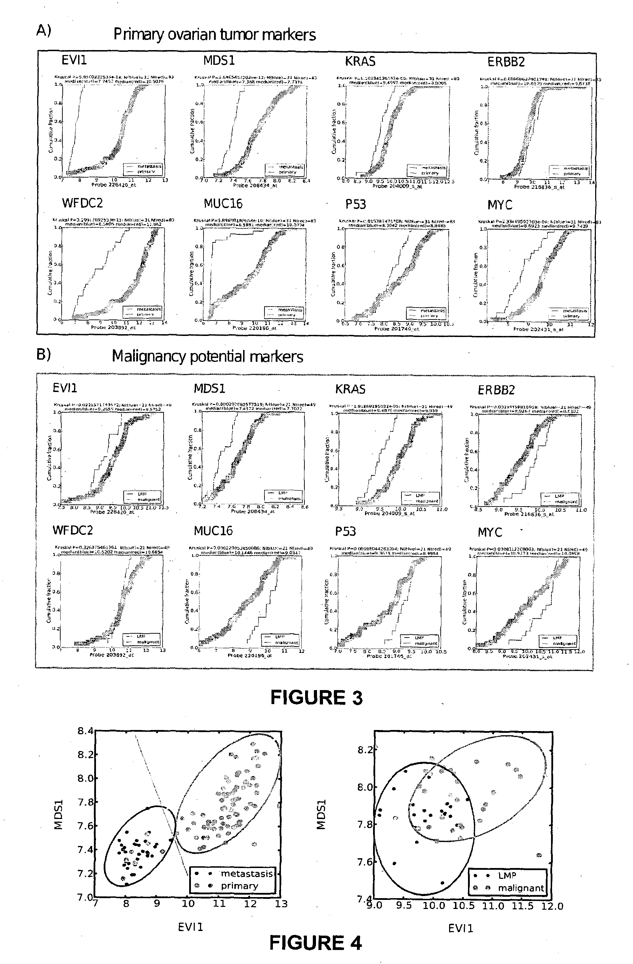

Figure 3 are graphs (A-B) showing that EVI1 and MDS1 genes of MECOM locus are more sensitive and specific than many conventional biomarkers in discriminating diagnosis of primary EOC tumors vs. breast cancer metastases in the ovaries (A) and LMP vs. malignant tumors (B). Cumulative distribution curves of microarray expression values are given.

Figure 4 are two graphs showing that a combination of expression levels of MECOM locus genes EVI1 and MDS1 is important for diagnostics of primary EOC tumors vs. breast cancer metastases in the ovaries (A) and LMP vs. malignant tumors (B). Microarray expression values are given.

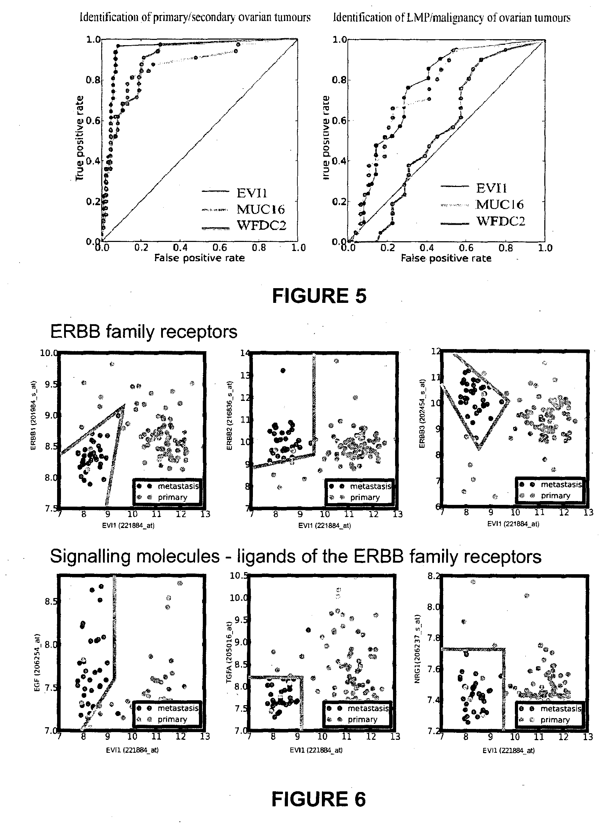

Figure 5 are graphs showing that MECOM locus gene EVI1 is a more sensitive and specific biomarker in of primary EOC tumors vs. breast cancer metastases in the ovaries (A) and LMP vs. malignant tumors (B) than the most sensitive and specific clinically used biomarkers WFDC2 and MUC16 which are amongst the many conventional biomarkers. ROC curves of microarray expression values are given.

Figure 6 are graphs showing that a combination of EVI1 and ERBB family genes expression are one of the most sensitive biomarkers of primary EOC. Microarray expression values are given.

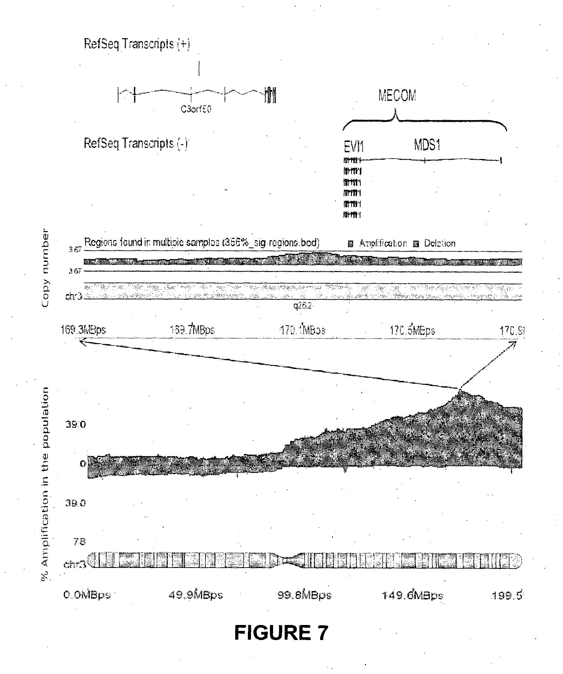

Figure 7 is an illustration of DNA copy number variations on chromosome 3 that shows that the 3' end of MECOM locus is the strongest gene amplification hot spot in the genomes of EOC tumor cells.

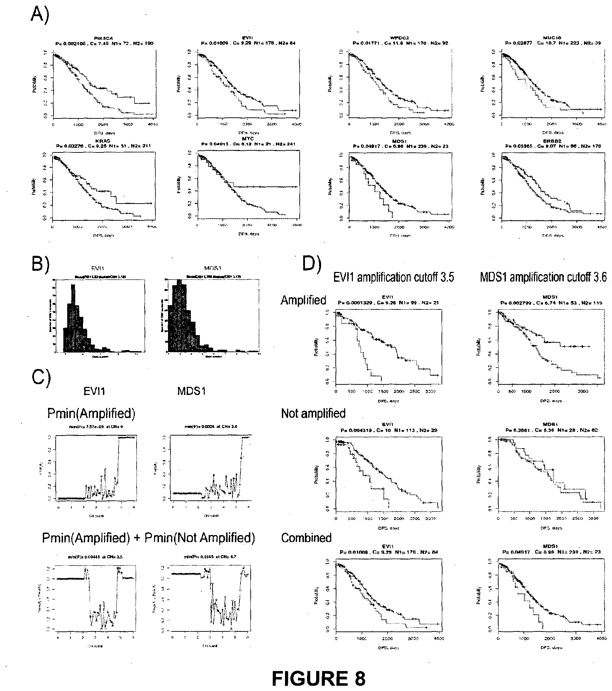

Figure 8 are graphs (A-D) showing A) that the amplification of MECOM locus together with the expression of its genes EVI1 and MDS1 are stronger indicators of EOC patients survival prognosis than many conventional biomarkers, B) the distribution of EVI1 and MDS1 genes copy number values, C) the optimal values $P_{min}$ of survival prognosis predicted by the combination of DNA copy number and expression values of EVI1 and MDS1, D) the optimal values giving the strongest survival prognosis are different for subgroups of patients with EVI1 and MDS1 gene amplification, the prognostic value of both genes being stronger within these patient subgroups.

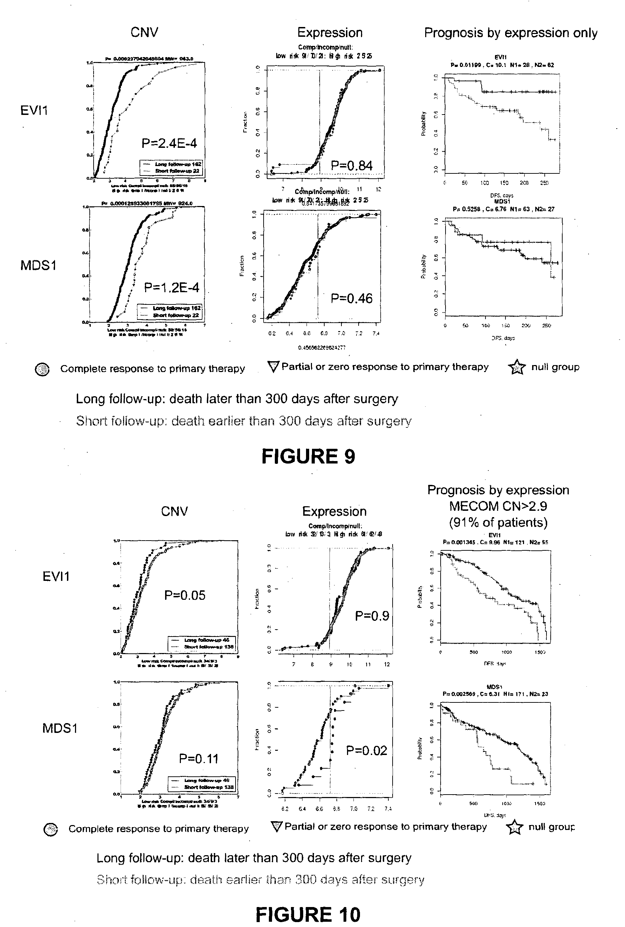

Figure 9 is a graph showing that EVI1 is a powerful marker for discriminating between poor and good prognosis patients at a short prognostic time period (up to 300 days after surgery treatment). Left to right: DNA copy number, microarray expression and survival curves of patients separated by the expression of EVI1 and MDS1 genes are presented.

Figure 10 are graphs that show that EVI1 is a marginally significant marker for discriminating between poor and good prognosis patients at medium prognostic time period (up to 1500 days after surgery treatment). Left to right: DNA copy number, microarray expression and survival curves of patients separated by the expression of EVI1 and MDS1 genes are presented.

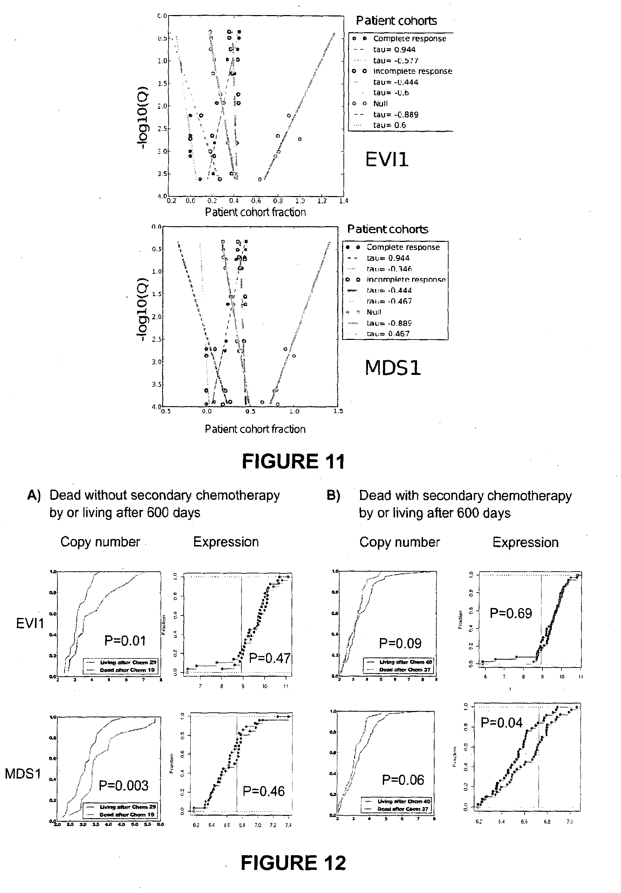

Figure 11 are nomograms showing the dependency of the primary therapy response outcome patient group composition by response cohort (patient cohort fraction) on EVI1 and MDS1 copy number quantile values Q. The nomograms are used for therapy outcome prediction. Linear equations are chosen to approximate the dependency, but other types of equation are also possible.

Figure 12 are graphs (A-B) showing a possibility of the secondary therapy outcome prediction based on EVI1 and MDS1 genes expression for A) patients without secondary chemotherapy B) with secondary chemotherapy. Copy number and microarray expression values are given.

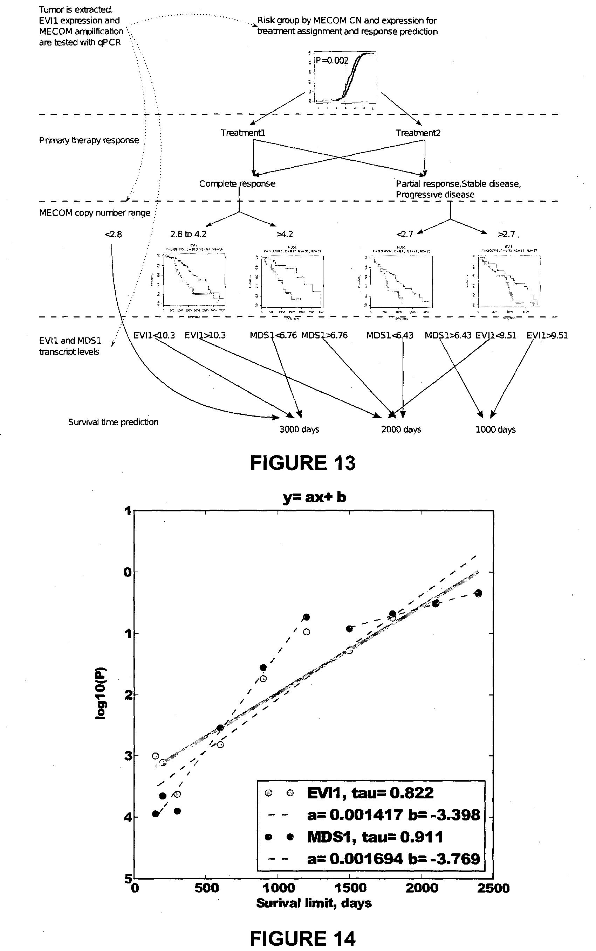

Figure 13 is an illustration of a method for robust survival prognosis using a combination of MECOM locus copy number and EVI1 and MDS1 expression values with primary therapy response outcome.

Figure 14 is a graph showing the dependence of P-value of the difference between MECOM copy number distributions for patients with good and poor prognoses for a given limited time period on the time limit with a strong positive correlation.

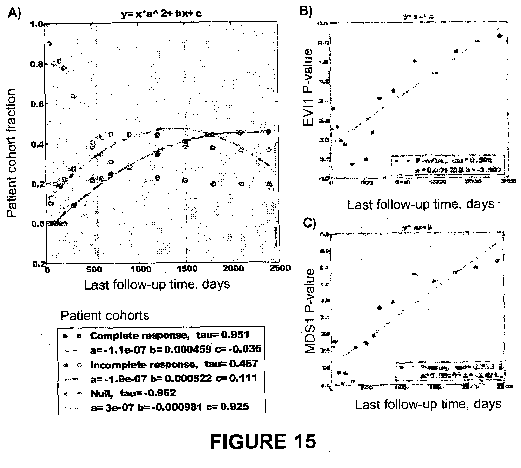

Figure 15 are graphs (A-C) showing that primary therapy response strongly correlates with patient last follow up time (A), which, in its turn, correlates with the P-values of EVI1 (B) and MDS1 (C) genes expression differentiating between patients with good and poor prognosis.

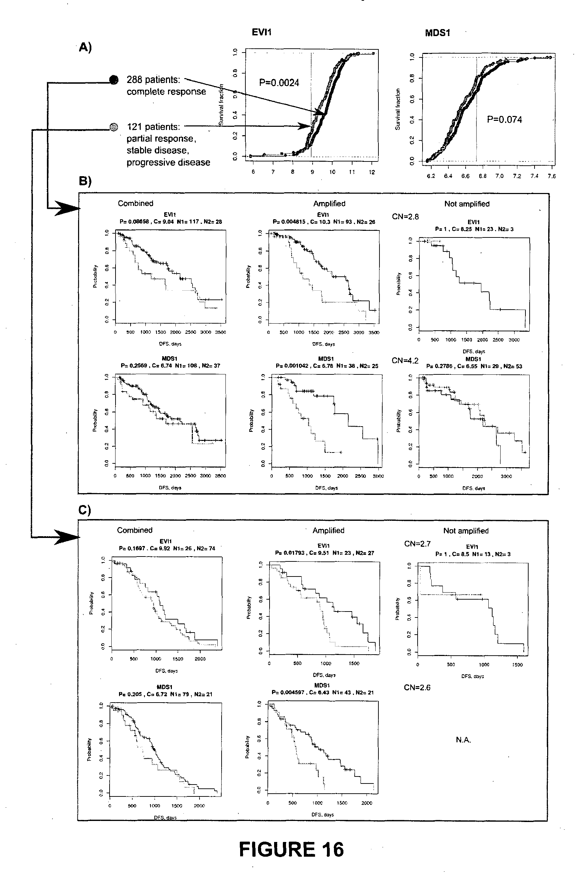

Figure 16 are graphs (A-C) showing that the EVI1 and MDS1 transcription is a significant prognostic factor when used with patients with complete response to the primary therapy ("Complete response" group) and compared with a combined group containing patients demonstrating partial response, stable or progressive disease ("Incomplete response"). A) The cumulative distribution functions of EVI1 and MDS1 gene expression values for "Complete response" (black dots) and "Incomplete response" (grey dots). B) That EVI1 and MDS1 copy number and expression values as survival prognostic markers for patients with complete response to primary therapy. C) EVI1 and MDS1 copy number (MECOM locus) and expression values as survival prognostic markers for patients with partial response to primary therapy, stable or progressive disease.

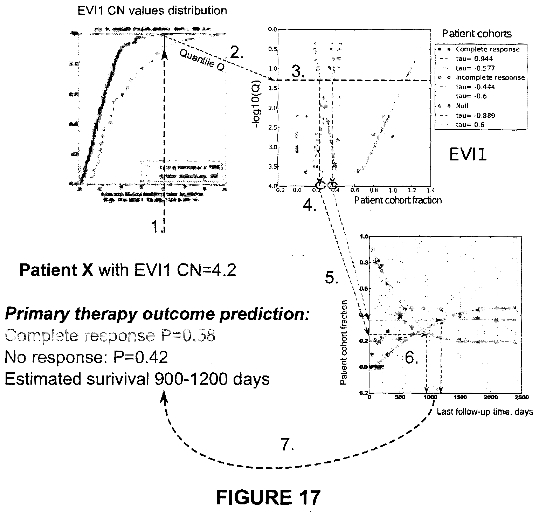

Figure 17 are graphs showing the algorithm for primary therapy outcome prediction and associated patient survival prognosis based on MECOM locus copy number data. 1) Identify the quantile (Q) of EVI1 gene copy number value, 2,3) determine the expected patient cohort composition based on Q, 4,5,6) determine the expected follow-up time for each patient cohort, 7) estimate the most probable survival time and the responses for each therapy outcome scenario (complete, partial or no response to the therapy).

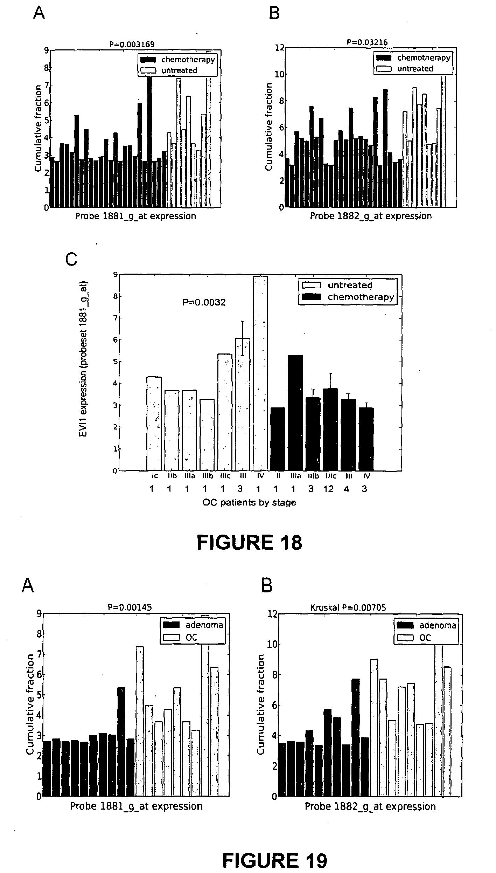

Figure 18 are graphs (A-C) showing that EVI1 expression is significantly different in tumors of the patients pre-treated with neo-adjuval chemotherapy, in comparison with tumors of patients untreated prior to surgery. Expression of EVI1 is measured by probesets 1881_g_at (A and C) and 1882_g_at (B). [HG_U95Av2 Alignments to Genome, PSL (2.3 MB, 9/28/06)]. As can be seen these results are significantly different in the tumors of the patients after neo-adjuval chemotherapy.

Figure 19 are graphs (A and B) showing that EVI1 expression is significantly different in benign (adenoma) and malignant (ovarian carcinoma) ovarian tumors. Expression of EVI1 is measured by probesets 1881_g_at (A) and 1882_g_at (B).

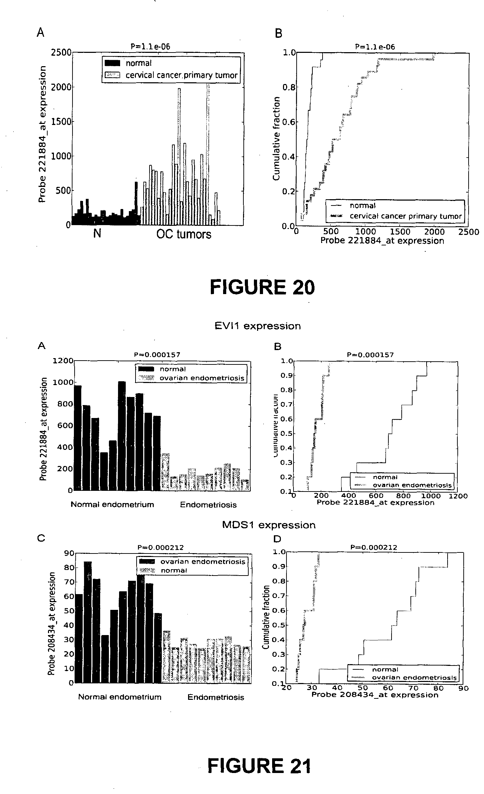

Figure 20 are graphs (A and B) showing that EVI1 expression is significantly different in cervical cancer tumors compared to normal cervical epithelium. For EVI1 (probeset 221884_at) expression values (A) and their cumulative distribution (B) are presented.

Figure 21 are graphs (A, B, C and D) showing that EVI1 and MDS1 expression is significantly different in endometriosis tumors compared to normal endometrium. A, B - EVI1 (probeset 221884_at) expression. C, D - MDS1 (probeset 208434_at) expression. The expression values (A, C) and EV71 and MDS1 cumulative distribution are presented as graphs B and D respectively.

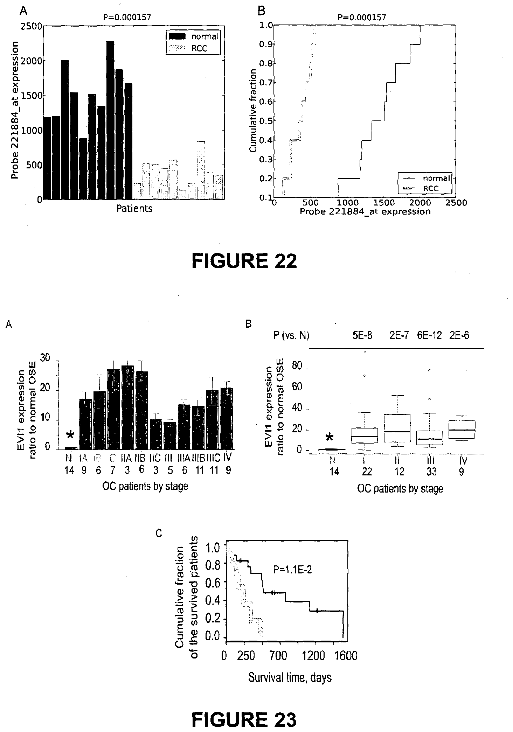

Figure 22 are graphs (A and B) showing that EVI1 expression is significantly different in clear cell renal cell carcinoma tumor, in comparison with normal kidney epithelium. For EVI1 (probeset 221884_at) expression values (A) and their cumulative distribution (B) are presented.

Figure 23 are graphs (A,B and C) showing that the primers (EVI1-For and EVI1-rev), by measuring EVI1 expression, can discriminate ovarian cancer tumor from ovarian surface epithelium with 100% specificity and sensitivity and can be used for patient survival prognosis. A) Distribution of EVI1 expression qPCR fold change (relative to normal ovarian surface epithelium, "N") measurements by ovarian cancer stage; B) Fold change distribution and the P-values comparing ovarian cancer tumors at each stage (I to IV) with ovarian surface epithelium (N); C) Cumulative fraction of surviving patients significantly discriminated by the EVI1 expression measurement with fold change cutoff 16.76; P-value for the Cox-proportional survival model is given; Thick grey line marks the survival of the unfavourable prognosis group with EVI1 expression higher than the cutoff value.

## DETAILED DESCRIPTION OF THE PREFERRED EMBODIMENTS

Bibliographic references mentioned in the present specification are for convenience listed in the form of a list of references and added at the end of the examples. The whole content of such bibliographic references is herein incorporated by reference.

## Definitions

For convenience, certain terms employed in the specification, examples and appended claims are collected here.

The term "aptamer" is herein defined to be oligonucleic acid or peptide molecule that binds to a specific target molecule. In particular, an aptamer used in the present invention may be generated using different technologies known in the art which include but is not limited to systematic evolution of ligands by exponential enrichment (SELEX) and the like. The term "comprising" is herein defined to be that where the various components, ingredients, or steps, can be conjointly employed in practicing the present invention. Accordingly, the term "comprising" encompasses the more restrictive terms "consisting essentially of" and "consisting of." With the term "consisting essentially of" it is understood that the method according to any aspect of the present invention "substantially" comprises the indicated step as "essential" element. Additional steps may be included.

The term "difference" between two groups of patients is herein defined to be the statistical significance (p-value) of a partitioning of the patients within the two groups. Thus, achieving a "maximum difference" means finding a partition of maximal statistical significance (i.e. minimal p-value).

The term "label" or "label containing moiety" refers in a moiety capable of detection, such as a radioactive isotope or group containing same and nonisotopic labels, such as enzymes, biotin, avidin, streptavidin, digoxygenin, luminescent agents, dyes, haptens, and the like. Luminescent agents, depending upon the source of exciting energy, can be classified as radio luminescent, chemiluminescent, bio luminescent, and photo luminescent (including fluorescent and phosphorescent). A probe described herein can be bound, for example, chemically bound to label-containing moieties or can be suitable to be so bound. The probe can be directly or indirectly labelled.

The term "locus" is herein defined to be a specific location of a gene or DNA sequence on a chromosome. A variant of the DNA sequence at a given locus is called an allele. The ordered list of loci known for a particular genome is called a genetic map. Gene mapping is the process of determining the locus for a particular biological trait. For example, the MECOM locus comprises at least two genes MDS1 and EVI1, which expression results in transcription of, at least two corresponding transcripts, i.e. mRNA variants. The two transcripts may be the longer transcript of MDS1 and the shorter transcript of EVI1. In particular, the sequence of the MECOM locus may comprise SEQ ID NO: 10.

The term "MECOM locus" is herein defined according to the definition provided in the RefSeq NCBI database as "MDS1 and EVI1 complex locus (MECOM)" or "MDS1 and EVI1 complex locus" (Unigene Hs.659873) and may be essentially characterized by its genomic coordinates hg18.chr3:170,283,981-170,864,257 (SEQ ID NO: 10) and by its non-limiting longest transcripts NM_004991.3) SEQ ID NO:4, NM_001205194.1 (SEQ ID NO:5), NM_001105078.3 (SEQ ID NO:11), NM_001105077.3 (SEQ ID NO:12), NM_005241.3 (SEQ ID NO:13), NM_001164000.1 (SEQ ID NO:14), NM_001163999.1 (SEQ ID NO:15) and the like. The sequences of these seven isoforms may be targeted by the probes according to any aspect of the present invention. The probes of the present invention may be capable of detecting these isoforms and other isoforms found in other cells.

The term "copy number (CN) value" or "DNA copy number value" is herein defined to refer to the number of copies of at least one DNA segment (locus) in the genome. The genome comprises DNA segments that may range from a small segment, the size of a single base pair to a large chromosome segment covering more than one gene. This number may be used to measure DNA structural variations, such as insertions, deletions and inversions occurring in a given genomic segment in a cell or a group of cells. In particular, the CN value may be determined in a cell or a group of cells by several methods known in the art including but not limited to comparative genomic hybridisation (CGH) microarray, qPCR, electrophoretic separation and the like. CN value may be used as a measure of the copy number of a given DNA segment in a genome. In a single cell, the CN value may be defined by discrete values (0, 1, 2, 3 etc.). In a group of cells it may be a continuous variable, for example, a measure of DNA fragment CN ranging around 2 plus/minus increment d (theoretically or empirically defined variations). This number may be larger than 2+d or smaller than 2-d in the cells with a gain or loss of the nucleotides in a given locus, respectively.

The term "complementary" is used herein in reference to polynucleotides (i.e., a sequence of nucleotides such as an oligonucleotide or a target nucleic acid) related by the base-pairing rules. For example, for the sequence "5'-A-G-T-3'," is complementary to the sequence "3'-T-C-A-5'." The degree of complementarity between nucleic acid strands has significant effects on the efficiency and strength of hybridization between nucleic acid strands. This is of particular importance in amplification reactions, as well as detection methods that depend upon binding between nucleic acids. In particular, the "complementary sequence" refers to an oligonucleotide which, when aligned with the nucleic acid sequence such that the 5' end of one sequence is paired with the 3' end of the other, is in "anti-parallel association." Certain bases not commonly found in natural nucleic acids may be included in the nucleic acids disclosed herein and include, for example, inosine and 7-deazaguanine. Complementarity need not be perfect; stable duplexes may contain mismatched base pairs or unmatched bases. Those skilled in the art of nucleic acid technology can determine duplex stability empirically considering a number of variables including, for example, the length of the oligonucleotide, base composition and sequence of the oligonucleotide, ionic strength and incidence of mismatched base pairs. Where a first oligonucleotide is complementary to a region of a target nucleic acid and a second oligonucleotide has complementary to the same region (or a portion of this region) a "region of overlap" exists along the target nucleic acid. The degree of overlap may vary depending upon the extent of the complementarity.

The term "comprising" is herein defined as "including principally, but not necessarily solely". Furthermore, the term "comprising" will be automatically read by the person skilled in the art as including "consisting of". The variations of the word "comprising", such as "comprise" and "comprises", have correspondingly varied meanings.

The term "derivative," is herein defined as the chemical modification of the oligonucleotides of the present invention, or of a polynucleotide sequence complementary to the oligonucleotides. Chemical modifications of a polynucleotide sequence can include, for example, replacement of hydrogen by an alkyl, acyl, or amino group.

The term "fragment" is herein defined as an incomplete or isolated portion of the full sequence of an oligonucleotide which comprises the active/binding site(s) that confers the sequence with the characteristics and function of the oligonucleotide. In particular, it may be shorter by at least one nucleotide or amino acid. More in particular, the fragment comprises the binding site(s) that enable the oligonucleotide to bind to influenza virus. A fragment of the oligonucleotides of the present invention may be about 20 nucleotides in length. In particular, the length of the fragment may be at least about 10 nucleotides in length. For example, the fragment of the forward primer may comprise at least 10, 12, 15, 18 or 19 consecutive nucleotides of SEQ ID NO:1, and/or the reverse primer may comprise at least 10, 12, 15, 18, 19, 20, 22, or 24 consecutive nucleotides of SEQ ID NO:2. More in particular, the fragment of the primer may be at least 15 nucleotides in length.

The term "mutation" is herein defined as a change in the nucleic acid sequence of a length of nucleotides. A person skilled in the art will appreciate that small mutations, particularly point mutations of substitution, deletion and/or insertion has little impact on the stretch of nucleotides, particularly when the nucleic acids are used as probes. Accordingly, the oligonucleotide(s) according to the present invention encompasses mutation(s) of substitution(s), deletion(s) and/or insertion(s) of at least one nucleotide. Further, the oligonucleotide(s) and derivative(s) thereof according to the present invention may also function as probe(s) and hence, any oligonucleotide(s) referred to herein also encompasses their mutations and derivatives. For example, if mutations occur at a few base positions at any primer hybridization site of the target gene, particularly to the 5'-terminal, the sequence of primers may not affect the sensitivity and the specificity of the primers.

With respect to associations between disease and CN value, a level of variation (deviation) in a DNA segment CN might be important. A level of positive or negative increment of the CN from normal dynamical range in a DNA sample of a given cell group or a single cell may be called CN variation.

The term "diagnosing" and "diagnosis" is herein defined to include the act or process of identifying the existence and/or type of cancer from which an individual may be suffering. Thus, in one embodiment, diagnosis includes the differentiation of a particular cancer type, namely EOC, from one or more other cancers. In an alternative embodiment, binding moieties of the invention are for use in classifying EOC patients into clinically relevant groups based on overall survival and/or cancer-specific survival.

The term "prognosis" is herein defined to include the act or process of predicting the probable course and outcome of a cancer, e.g. determining survival probability and/or recurrence-free survival (RFS) probability.

The term "binding moiety" is herein defined to be a molecule or entity that is capable of binding to target genomic DNA, a target protein or mRNA encoding the same. For example, a binding moiety can be a probe such as a single stranded oligonucleotide at the time of hybridization to a target protein. Probes include but are not limited to primers, i.e., oligonucleotides that can be used to prime a reaction, for example at least in a PCR reaction. In particular, the probe may be capable of binding to ENV1 or MDS1 protein or mRNA encoding the same and be used to quantify the gene expression level of ENV1 or MDS1. In another embodiment, the probe may capable of binding to the genomic DNA of MECOM locus (i.e. ENV1 or MDS1 genomic DNA) and may be capable of determining the copy number of ENV1 or MDS1.

It will be appreciated by persons skilled in the art that the binding moieties of the invention may be used for the diagnosis or prognosis of EOC of any histological subtype (for example, serous, mucinous, endometrioid, clear cell, undifferentiated or unclassifiable).

The term "sample" is herein defined to include but is not limited to be blood, sputum, saliva, mucosal scraping, tissue biopsy and the like. The sample may be an isolated cell sample which may refer to a single cell, multiple cells, more than one type of cell, cells from tissues, cells from organs and/or cells from tumors.

A person skilled in the art will appreciate that the present invention may be practiced without undue experimentation according to the method given herein. The methods, techniques and chemicals are as described in the references given or from protocols in standard biotechnology and molecular biology text books.

According to one aspect, the present invention provides an *in vitro* method for obtaining information in relation to a medical condition in a subject, the method comprising determining in a sample of the subject:

(i) gene expression level of at least one gene in the MECOM locus, selected from *EVI1* and *MOS1;* and/or
(ii) copy number of at least one gene in the MECOM locus selected from *EVI1* and *MOS1;*

wherein the information is selected from the group consisting of:

a) whether the subject has epithelial ovarian cancer, preferably high grade serous ovarian carcinoma and/or a predisposition to epithelial ovarian cancer, preferably high grade serous ovarian carcinoma, wherein the gene expression level against at least one statistically optimized gene expression cutoff value derived from a plurality of training subjects with known diagnosis and/or the at least one gene copy number against at least one statistically optimized gene copy number cutoff value derived from a plurality of training subjects with known diagnosis is/are indicative of said information, wherein said optimized cutoff value is obtained with the following steps;

(i) extract the expression level or copy number level of a gene for each training subject;
(ii) obtain the cumulative distribution function of the gene expression or copy number levels of two sets of training subjects, one set being associated with a positive diagnosis and one set being associated with a negative diagnosis; and
(iii) select a cut-off value which divides the training subjects into two groups, based on whether the gene expression level or copy number level of each training subject is higher or lower than the cut-off value, and which minimizes the false positive and/or false negative rate in the diagnosis using one-dimensional statistical optimization;
or, if the expression and the copy number values are identified in the same sample, the optimal cutoff for both expression and copy number are obtained simultaneously by minimising the false positive and/or false negative rates using two-dimensional statistical optimization;

wherein said epithelial ovarian cancer determination comprises using PCR primers comprising a forward primer SEQ ID NO: 1 and reverse primer SEQ ID NO: 2 and probe SEQ ID NO: 3, or using at least one of the probe sets selected from 1881_g_at, 1882_g_at, 208434_at, 221884_at and 226420_at;

b) survival prognosis of the subject when the subject has epithelial ovarian cancer, preferably high grade serous ovarian carcinoma, wherein the expression level against at least one statistically optimized gene expression cutoff value and/or the at least one gene copy number against at least one statistically optimized gene copy number cutoff value is/are indicative of said information, wherein, for primary patient stratification, the optimal copy number and expression cutoff values are obtained for each gene using a two-step optimization algorithm as follows;

(i) extract the expression level and copy number of the gene for each training subject;
(ii) statistically estimate a copy number cutoff value and divide the training subjects into two cohorts according to whether the copy number of the gene is above or below the estimated copy number cutoff value; and
(iii) for each said cohort, select a gene expression cutoff value that divides the training subjects in each cohort into two groups based on whether the gene expression level of each training subject is higher or lower than the expression cutoff value, wherein the cutoff value minimizes the Wald test P-value of Kaplan-Meier survival models;

wherein said survival prediction determination comprises using PCR primers comprising a forward primer SEQ ID NO: 1 and reverse primer SEQ ID NO: 2 and probe SEQ ID NO: 3, or using at least one of the probe sets selected from 1881_g_at, 1882_g_at, 221884_at and 226420_at;

c) effectiveness of treatment of epithelial ovarian cancer, preferably high grade serous ovarian carcinoma, in the subject, wherein the gene expression level against at least one statistically optimized gene expression cutoff value and/or the at least one gene copy number against at least one optimized gene copy number cutoff value are indicative of said information;

wherein said effectiveness of treatment of epithelial ovarian cancer determination comprises using PCR primers comprising a forward primer SEQ ID NO: 1 and reverse primer SEQ ID NO: 2 and probe SEQ ID NO: 3, or using at least one of the probe sets selected from 1881_g_at, 1882_g_at, 221884_at and 226420_at;

d) whether epithelial ovarian cancer, preferably high grade serous ovarian carcinoma, in the subject is of primary or secondary origin, wherein the gene expression level against at least one statistically optimized gene expression cutoff value and/or the at least one gene copy number against at least one statistically optimized gene copy number cutoff value are indicative of said information;

wherein said determination of whether the epithelial ovarian cancer is of primary or secondary origin comprises using PCR primers comprising a forward primer SEQ ID NO: 1 and reverse primer SEQ ID NO: 2 and probe SEQ ID NO: 3, or using at least one of the probe sets selected from 226420_at, 221884_at and 208434_at; and

e) whether the subject has high grade serous ovarian carcinoma, cervical cancer, ovarian endometriosis, clear cell renal carcinoma and/or a predisposition to cervical cancer, ovarian endometriosis, clear cell renal carcinoma, wherein the level against at least one statistically optimized gene expression cutoff value and/or the at least one gene copy number against at least one statistically optimized gene copy number cutoff value are indicative of the subject having high grade serous ovarian carcinoma, cervical cancer, endometriosis, clear cell renal carcinoma and/or a predisposition to cervical cancer, endometriosis, or clear cell renal carcinoma;

wherein said determination of the type of cancer comprises using PCR primers comprising a forward primer SEQ ID NO: 1 and reverse primer SEQ ID NO: 2 and probe SEQ ID NO: 3, or using at least one of the probe sets selected from 226420_at, 1881_g_at, 1882_g_at, 221884_at and 208434_at,

wherein said gene expression level in a)-e) is determined from at least one transcript variant or isoform comprising a nucleic acid sequence selected from the group consisting of SEQ ID NO: 4, SEQ ID NO: 5, SEQ ID NO: 11, SEQ ID NO: 12, SEQ ID NO: 13, SEQ ID NO: 14 and SEQ ID NO: 15.

Embodiments are provided by the dependent claims.

According to a second aspect, there is provided the use of specific oligonucleotides,derived from one or more of the individual probe sets selected from the group consisting of 1881_g_at, 1882_g_at, 208434_at; 221884_at and 226420_at, their fragments, derivatives, mutants and complementary sequences thereof, in a method according to the first aspect.

The present invention also provides the use of a kit comprising a forward amplification primer having a sequence comprising SEQ ID NO: 1, a reverse amplification primer having a sequence comprising SEQ ID NO: 2 and a probe having a sequence comprising SEQ ID NO: 3 or specific oligonucleotides derived from one or more of the individual probe sets selected from the group comprising 1881_g_at, 1882_g_at, 208434_at; 221884_at and 226420_at, their fragments, derivatives, mutants and complementary sequences thereof in a method for obtaining information in relation to a medical condition in a subject according to the method of the first aspect.

Also provided is a set of oligonucleotides consisting of,

a forward amplification primer having a nucleic acid sequence comprising SEQ ID NO: 1, a reverse amplification primer having a nucleic acid sequence comprising SEQ ID NO: 2 and a probe having a nucleic acid sequence comprising SEQ ID NO: 3,

wherein the set is capable of simultaneous detection and discrimination of complete protein-coding specific isoforms of EVI1 isoform only, MDS1 only and the combination of EVI1 and EVI1/MDS1 isoforms as defined by SEQ ID NO: 4, SEQ ID NO: 5, SEQ ID NO: 11, SEQ ID NO: 12, SEQ ID NO: 13, SEQ ID NO: 14 and SEQ ID NO: 15, and complete determination of transcription status of whole MECOM locus in an individual patient sample.

According to one related aspect, there is described at least one method for diagnosing n cancer, and/or predisposition to cancer in a subject, determining survival prognosis of a subject with cancer, and/or determining the effectiveness of treatment of cancer, and/or determining if the cancer is in a subject is of primary origin or secondary origin, the method comprising determining in a sample of the subject:

(i) gene expression level of at least one gene in the MECOM locus; and/or
(ii) copy number of at least one gene in the MECOM locus

wherein the gene expression level and/or copy number against at least one expression and copy number cutoff value respectively is indicative of the subject having cancer or predisposition to cancer and/or survival prognosis of a subject with cancer, and/or determining the effectiveness of treatment of cancer.

In particular, the EOC may be adenocarcinoma or malignant ovarian cancer. In particular, the MECOM locus expression and/or copy number may be used for differential diagnostics of ovarian cancer compared to other types of cancers and/or normal tissues.

According to one related aspect, there is provided at least one method for diagnosing epithelial ovarian cancer (EOC), predisposition to EOC in a subject, determining survival prognosis of a subject with EOC, and/or determining the effectiveness of treatment of EOC, and/or determining if an epithelial ovarian cancer in a subject is of primary origin or secondary origin, the method comprising determining in a sample of the subject:

(i) gene expression level of at least one gene in the MECOM locus; and/or
(ii) copy number of at least one gene in the MECOM locus

wherein the gene expression level and/or copy number against at least one expression and copy number cutoff value respectively is indicative of the subject having EOC or predisposition to EOC and/or survival prognosis of a subject with EOC, and/or determining the effectiveness of treatment of EOC.

EVI1 expression as a clinical biomarker may have the largest sensitivity and specificity among conventional biomarkers in the diagnostics of ovarian tumor metastasis. The EVI1 gene expression level is a good indicator of whether the tumors are primary or secondary. EVI1 gene expression may also be useful as a clinical biomarker with high specificity but lower sensitivity in the diagnostics of ovarian tumor malignancy potential.

In particular, EVI1 expression may be a clinical biomarker with large sensitivity and specificity among conventional biomarkers in the diagnostics of ovarian tumor primary origin and as a clinical biomarker with high specificity but lower sensitivity in the diagnostics of ovarian tumor malignancy potential.

The method according to the present invention is *in vitro,* where the steps are carried out on a sample isolated from the subject. The sample may be taken from a subject by any method known in the art. For example but not limiting, ovarian tumor material may be extracted from ovaries, fallopian tubes, uterus, vagina and the like. Metastatic tumor may be extracted from peritoneal cavity, other body organs, tissues and the like. Cancer cells may be extracted from non limiting examples such as biological fluids, which include but are not limited to peritoneal liquid, blood, lymph, urine, products of body secretion and the like.

Quantifying of expression ecotropic virus integration site 1 protein homolog (EVI1), Myelodysplasia syndrome-1 (MDS1) and other gene transcripts used according to any aspect of the present invention may be done using any technique of gene expression quantification. Such techniques include, but are not limited to quantitative PCR, semi-quantitative PCR, gene expression microarray, next generation RNA sequencing and the like.

The copy number of MECOM, EVI1, MDS1 and/or other genes used according to any aspect of the present invention may be determined using any technique of gene copy number quantification. Such techniques include, but are not limited to quantitative PCR, semi-quantitative PCR, SNP microarrays, next-generation sequencing, cytogenetic techniques (such as in-situ hybridization, comparative genomic hybridization, comparative genomic hybridization), Southern blotting, multiplex ligation-dependent probe amplification (MLPA) and Quantitative Multiplex PCR of Short Fluorescent Fragments (QMPSF) and the like.

In particular, the method according to the present invention comprises, consists of or consists essentially of determining the level of gene expression and/or copy number of the MECOM locus.

In particular, the method according to any aspect of the present invention comprises, consists of or consists essentially of determining the level of gene expression of EV1 and/or MDS1 of the MECOM locus. More in particular, the method further comprises, consists of or consists essentially of the step of determining the copy number of EV1 and/or MDS1 of the MECOM locus.

According to one embodiment, the method comprises, consists of or consists essentially of determining the level of gene expression and copy number of EV1 or MDS1. According to another embodiment, the method comprises, consists of or consists essentially of determining the level of gene expression of EV1 and copy number of MDS1. According to a further embodiment, the method comprises, consists of or consists essentially of determining the level of gene expression of MDS1 and the copy number of EV1. According to one embodiment, the method comprises, consists of or consists essentially of determining the level of gene expression of EV1 and MDS1 and the copy number of EV1. According to a further embodiment, the method comprises, consists of or consists essentially of determining the level of gene expression of MDS1 and the copy number of EV1 and MDS1. According to an even further embodiment, the method comprises, consists of or consists essentially of determining the level of gene expression and copy number of both EV1 and MDS1.

The method according to any aspect of the present invention may include a further step of determining the gene expression level of at least one further gene selected from the group consisting of MUC16, WFDC2, P53, KRAS, ERBB1, ERBB2, ERBB3, EGF, NGR1, TGFA, and MYC in the sample.

MUC16 and WFDC2 are existing clinically-approved markers for ovarian cancer. The use of these genes with EVI1 gene and/or the MDS1 may result in more sensitive and specific diagnosis and/or prgonosis. In particular, better results may be obtained with a combination of the EVI1 gene expression level and the WFDC2 gene expression level (i.e. double combination). This combination can be used to obtain more accurate information about a subject relating to ovarian cancer (for example, whether the ovarian epithelia of the subject is normal or cancerous). Alternatively, the EVI1 gene expression level may also be combined with both the WFDC2 gene expression level and the MUC16 gene expression level to achieve even more accurate results. This triple combination achieves a more specific gene signature (i.e. a more specific marker) for ovarian cancer as compared to the double combination or to using the EVI1 gene expression level alone.

The treatment may be selected from the group consisting of chemotherapy, surgery and post-surgery chemotherapy.

The combination of expression data of MDS1 with EVI1 could be used also in pair for increasing the discrimination ability of tumor subtypes in ovarian cancer for example but not limited to low malignancy potential tumors, breast cancer metastases in the ovary, EOC and the like.

The expression cutoff values and copy number cutoff values are obtained during a training stage using a plurality of training subjects with known diagnosis relating EOC whereby the training subjects comprise two sets of training subjects, each set associated with a different diagnosis. This may be done using a variety of ways.

In one example, the expression cutoff value is obtained for each gene with the following steps:

(1) extract the expression level of the gene for each training subject;

(2) obtain the cumulative distribution function of the gene expression levels of each set of training subjects (each set being associated with a different diagnosis as mentioned above); and

(3) select an expression cutoff value which divides the training subjects into two groups based on whether the gene expression level of each training subject is higher or lower than the expression cutoff value.

Any method known in the art for obtaining the cumulative distribution factor in step (2) may be used. In particular, the method assumes that there are two training sets: one with negative diagnosis/prognosis/prediction of size $N_1$, and the other with positive diagnosis/prognosis/prediction of size $N_2$, $X_1$ - the value of parameter $X$ for the negatively diagnosed patients, $X_2$ - the value of parameter $Y$ for the positively diagnosed patients, $C$ - the chosen cutoff level for parameter $X$.

Definitions:

$$CDF_X(x) = count(X \leq x)/N$$

$$\text{Observed negatives} = count(X \leq C)$$

$$\text{Observed positives} = count(X > C)$$

Then,

$$\begin{cases} TN = \text{True negatives} = count(X_1 \leq C) = N_1 CDF_{X_1}(C) \\ TP = \text{True positives} = count(X_2 \geq C) = N_2 CDF_{X_2}(C) \\ FN = \text{False negatives} = count(X_1 > C) = N_1(1 - CDF_{X_1}(C)) \\ FP = \text{False positives} = count(X_2 < C) = N_2(1 - CDF_{X_2}(C)) \end{cases}$$

In the simplest 1-dimensional case, the maximal difference is obtained with

$$C_{opt} = \underset{C}{argmin}(FP + FN) = \underset{C}{argmin}\left(N_1(1 - CDF_{X_1}(C)) + N_2(1 - CDF_{X_2}(C))\right)$$

In a more complex, 2-dimentional case (e.g. 2 genes A and B), the CDF becomes a 2-D function $CDF_X (X_A, X_B)$ and the maximal difference is obtained with a 2-D minimization.

In particular, the division of the training subjects into the two groups based on whether the gene expression level of each training subject is higher or lower than the expression cutoff value may be identical to a division of the training subjects into the two sets with different diagnoses. However, it may be difficult to achieve this ideal situation.

Therefore, in one example, the expression cutoff value may be chosen such that it achieves a maximum difference between the two divisions. In this example, the expression cutoff value may be chosen to minimize the number of false negatives. Depending on the diagnosis to be determined using the expression cutoff value, the false negatives can refer to the training subjects wrongly classified by the expression cutoff value into the group corresponding to the set of training subjects not having cancer, having secondary tumors, having low malignancy potential tumors and having a survival time beyond a certain value.

In another example, the diagnosis relates to the survival time of the subject and the cutoff expression value is chosen in step (3) to separate the training subjects into the two groups such that the two groups have maximally different (Wald statistics) survival curves (Cox-proportional model). This single 1-D optimization to achieve maximally different survival curves was used to obtain the expression cutoff values for the curves in Figure 8A and the curves for the "Combined" cohort in Figure 8D. The expression cutoff values for these figures are in log2 scale and are denoted by the letter "C" above the graphs.

In this study, data regarding the test subjects and training subjects are obtained from the same database with the test subjects having known diagnosis as well (though, this known diagnosis is not required for obtaining information about the test subject). Note that it cannot be guaranteed that an expression cutoff value obtained using a group of subjects (training subjects) can definitely be used for evaluating another group of subjects (test subjects). Neither can it be guaranteed that the expression cutoff value can be used in a general diagnostic kit for all test subjects. It has been demonstrated through years of clinical practice that there is almost always a specific cohort having clinical values (obtained for a specific drug or marker) significantly different from the clinical values obtained during clinical trials. Many existing and certified diagnostic/prognostic kits (e.g. MammaPrint, OncoMine) face the same problem. To determine a more optimal cutoff expression value, either more specific to a given cohort, or more powerful across several cohorts, specialized clinical studies may be required.

In one example, a copy number cutoff value (for primary patient stratification) and two expression cutoff values are obtained for each gene using a two-step optimization algorithm as follows:

(1) extract the expression level and copy number of the gene for each training subject;

(2) estimate a copy number value and divide the training subjects into two cohorts according to whether their copy numbers of the gene is above or below the copy number;

(3) for each cohort obtained in step (2), select an expression value that divides the training subjects into two groups based on whether the gene expression level of each training subject is higher or lower than the expression value. In another example, the diagnosis relates to the survival time of the subject and the expression value is chosen to separate the training subjects in the cohort into two groups such that the two groups have maximally different (Wald statistics) survival curves (Cox-proportional model). The training subjects in each cohort also comprise two sets of training subjects, each set associated with a different diagnosis. In a second example, the expression value is selected such that it achieves a maximum difference between the division of the training subjects in the cohort into the two groups based on the expression value and a division of the training subjects in the cohort into the two sets with different diagnoses. For example, the expression value may be chosen to minimize the number of false negatives. This way of selecting the expression value can be used for any type of diagnosis, include a diagnosis relating to the survival time of the subject.

(4) repeat steps (2) - (3) for a range of copy numbers;

(5) select the copy number cutoff value that achieves the maximum difference between the survival curves in a cohort (as in the first example) or the maximum difference between the divisions in a cohort (as in the second example). In one example, only the cohorts with the copy number of the gene above the respective estimated copy numbers are considered for selecting the copy number cutoff value. The expression cutoff values are then obtained as the expression values associated with the copy number cutoff value.

The cutoff values for obtaining the graphs (corresponding to both the "Amplified" and "Not amplified" cohorts) in Figure 8D are obtained using the two-step optimization algorithm above.

The copy number of the gene of the subject may be determined from a tumor of the subject. The method may be for determining survival prognosis of the subject, wherein the method further comprises the steps of:

(i) parametrizing a dependence between a patient cohort fraction and the copy number of the gene of the set training subjects;

(ii) parametrizing a dependence between the patient cohort fraction and the survival time of the set of training subjects; and

(iii) using the copy number of the subject to determine the patient cohort fraction from the dependence of (i) and using the patient cohort fraction of the subject to determine an estimated survival time of the subject using dependence of (ii).

The survival time of the training subject may be based on a last follow-up time for the training subject. According to any aspect of the present invention, last follow-up time may refer to the last visit by the subject to a medical practitioner to be examined. It may be 50, 100, 300, 500, 1000, 1500, 2000, 2500 and the like from prognosis of EOC.

The method may be for determining effectiveness of treatment further comprises the steps of:

(i) parametrizing a dependence between the copy number of the gene of the training subject and a possible treatment response of the training subject selected from a group consisting of:

- a complete response;

- an incomplete response; and

- a null response; and

(ii) using the copy number of the subject to determine an estimated possible treatment response of the subject.

According to one aspect, there is provided at least one kit for diagnosing epithelial ovarian cancer and/or predisposition to epithelial ovarian cancer in a subject, the kit comprising at least one probe that can identify the level of expression and/or copy number of at least one gene in the MECOM locus. The probe may be an oligonucleotide, aptamer, antibody and/or drug that may bind to MDS1and/or EVI1 gene/protein suitably.

The probe could be a drug. A non-limiting example of the drug may be sinefungin (Sigma-Aldrich), deazaneplanocin (Moravek Biochemicals) other inhibitors of histone-methytransferases and their derivatives and the like.

According to a further related aspect, there is provided at least one computer system having a processor arranged to perform a method according to any aspect of the present invention.

According to another related aspect, there is provided a computer program product such as a tangible data storage device, readable by a computer and containing instructions operable by a processor of a computer system to cause the processor to perform a method according to any aspect of the present invention.

According to a further related aspect, there is provided a nucleotide sequence selected from the group consisting of SEQ ID NOs: 1-3, fragments, derivatives, variants and complementary sequences thereof. In particular, EVI1-For and EVI1-Rev are nucleotide sequences of a pair of primers suitable for PCR-based techniques (including, but not limited to quantitative PCR), which may specifically measure expression of EVI1 transcript. Forward primer (termed EVI1-For) sequence 5'-GGTTCCTTGCAGCATGCAAGACC-3' (SEQ ID NO:1). Reverse primer sequence (termed EVI1-Rev) 5'-GTTCTCTGATCAGGCAGTTGG (SEQ ID NO:2). EVI1-Flu is a nucleotide sequence of a fluorescent probe- FAM6-TACTTGAGGCCTTCTCCAGG-TAMRA (SEQ ID NO:3), which can specifically measure expression of EVI1 transcript. These seqeunces may be capable of detecting any EVI1 isoform in a sample. In particular, these sequences may be capable of detecting a small quantity of the EVI1 isoform in the sample.

The primer sequences may comprise, consists of or consists essentially of the sequences of SEQ ID NO:1, 2 or 3. Having now generally described the invention, the same will be more readily understood through reference to the following examples which are provided by way of illustration, and are not intended to be limiting of the present invention.A person skilled in the art will appreciate that the present invention may be practised without undue experimentation according to the method given herein. The methods, techniques and chemicals are as described in the references given or from protocols in standard biotechnology and molecular biology text books.

## EXAMPLES

Standard molecular biology techniques known in the art and not specifically described were generally followed as described in Sambrook and Russel, Molecular Cloning: A Laboratory Manual, Cold Springs Harbor Laboratory, New York (2001).

## Clinical data description

The National Institute of Health (NIH) Cancer Genome Atlas (TCGA) database (McLendon, 2008) was used for copy number analysis of patients with ovarian cancer. The data on 514 EOC patients were downloaded from TCGA website. Among them 449 (87%) patients were initially classified to stages 3 and 4 of EOC; 448 (87%) patients received chemotherapy; 44 (8.5%) patients were younger than 45 years old, 21 (4%) were between 45 and 65 years old and 290 (56%) were older than 65. For 484 (94%) patients the time of the last follow-up and for 264 (51%) the time of death were defined.

For copy number analysis the data on 504 patients (of 514 in total) in TCGA set were available. Among them for 337 samples gene expression data was also available. This group of samples was used for survival analysis.

The predictive power of MECOM transcripts expression and gene copy number on the efficiency of treatment on 524 patients from TCGA database was tested. The patients were stratified by the type of response to primary therapeutic treatment into 2 classes: 1) 288 patients with complete response, 2) 121 patients with "incomplete response" (including partial response, stable or progressive disease), and 3) 115 patients with no response registered ("null response"), largely due to their deaths earlier than the due time of primary therapy response assessment.

For gene expression analysis the following publicly available datasets were obtained from Gene Expression Omnibus (GEO) website (Edgar R. 2002), GSE12172 including 90 samples (Anglesio MS, 2008), GSE20656 including 172 samples (Meyniel JP, 2010), GSE14407 including 24 patients (Bowen NJ, 2009). Among the 72 patients of GSE12172 dataset, which passed the quality assessment, all the tumors were characterized with serous phenotype, 56 (77%) were classified to stages 3 and 4 of EOC, 50 (72%) tumors were characterized as malignant, 22 (28%) were characterized as LMP (low malignancy potential).

Among the 116 patients of GSE20656 dataset, which passed the quality assessment, 59 tumors (78% of of the 76 tumors with available information) were characterized with serous phenotype, 55 (68% of the 81 tumors with available information) were classified to stages 3 and 4 of EOC, 83 (72%) tumors were identified as primary EOC, 28 (72%) were identified as breast cancer metastases in the ovaries. Among the 24 patients of GSE14407 12 patients were diagnosed with EOC at stages Ic (2 patients), II and IIb (2 patients), III (1 patient), IIIc (5 patients), IV (2 patients). The rest 12 samples were taken from normal ovarian epithelium of patients with subjected to surgical treatment if diseases different from epithelial cancer. Among the 43 ovarian cancer patients of GSE7463 dataset 33 were diagnosed with malignant ovarian carcinomas and 10 with non-malignant adenomas (Moreno CS et al., 2007). Among the malignant ovarian carcinoma patients 24 received neo-adjuvant chemotherapy prior to surgery and 9 did not receive it.

From GSE9750 microarray data set, expression values of 33 primary cervical cancer tumors and 24 samples from normal cervical epithelium were used (Scotto L et al, 2008). Dataset GSE7305 contained data featuring 10 ovarian endometrium tumors and 10 normal endometrium samples (Hever A et al, 2007). From GSE6344 10 clear cell renal cell carcinoma tumors (5 stage I and 5 stage II) and 10 normal kidney epithelium samples were used (Gumz ML et al, 2007).

Detailed information on the studied clinical cohorts can be found in Tables 1 and 2 below.

Table 1. Ovarian carcinoma vs LMP

| Parameter | Value | | No. of Patients |
|---|---|---|---|
| **GEO** | **GSE12172** | | **72** |
| **Primary.Site** | **NA** | | **72** |
| **Source Name** | **OVARIAN CARCINOMA** | | **50** |
| | **OVARIAN TUMOR(LMP)** | | **22** |
| | | | |
| Grade | | 2 | 11 |
| | | 3 | 37 |
| | HIGH | | 10 |
| | LOW | | 8 |
| | NA | | 6 |
| stage1 | | 1 | 11 |
| | | 2 | 5 |
| | | 3 | 47 |
| | | 4 | 9 |
| stage2 | A | | 14 |
| | B | | 6 |
| | C | | 42 |
| | NA | | 10 |
| histotype | SEROUS | | 72 |
| **tumor/metastasis** | **METASTASIS** | | **14** |
| | **PRIMARY** | | **58** |
| **LMP/malignant** | **LMP** | | **22** |
| | **MAUGNANT** | | **50** |
| subtype 2 | MUTANT BRAF | | 9 |
| | MUTANT ERBB2 | | 2 |
| | MUTANT KRAS | | 10 |
| | NOT MUTATION TESTED | | 18 |
| | WILD TYPE (BRAF/KRAS) | | 27 |
| | WILD TYPE (BRAF/KRAS/ERBB2) | | 6 |
| Micropapillary feature | NA | | 54 |
| | NEGATIVE | | 9 |

(continued)

| Parameter | Value | No. of Patients |
|---|---|---|
| (LMP) implants | POSITIVE | 9 |
| | MICRO-INVASIVE | 3 |
| | NA | 54 |
| | NEGATIVE | 6 |
| | NON-INVASIVE | 9 |
| Co-exhistant histology | ADJACENT BRENNER TUMOR | 1 |
| | ADJACENT INTRAEPITHELIAL CARCINOMA | 1 |
| | ADJACENT LMP | 2 |
| | ADJACENT LMP/MPSC | 1 |
| | ADJACENT SEROUS CYSTADENOMA | 1 |
| | NA | 56 |
| | NO NOTED | 10 |
| Cluster info | INV CLUSTER | 48 |
| | LMP CLUSTER | 24 |
| arrayed site | OV | 58 |
| | PE | 14 |

Table 2 Ovarian carcinoma vs. Breast cancer metastasis

| | Value | | No. of Patients |
|---|---|---|---|
| **GEO** | **GSE20565** | | **116** |
| **Primary.Site** | **OVARY** | | **116** |
| **Source Name** | **BREAST METASTASIS IN THE OVARY** | | 27 |
| | **OVARIAN CARCINOMA** | | 76 |
| | **PLAUSIBLE BREAST METASTASIS IN TH** | | 7 |
| | **PLAUSIBLE OVARIAN CARCINOMA** | | 6 |
| Grade | | 1 | 6 |
| | | 2 | 24 |
| | | 3 | 53 |
| | NA | | 33 |
| stage 1 | | 1 | 18 |
| | | 2 | 8 |
| | | 3 | 44 |
| | | 4 | 11 |
| | NA | | 35 |
| stage2 | A | | 14 |
| | B | | 9 |
| | C | | 47 |
| | NA | | 46 |
| histotype | ADENOCARCINOMA | | 2 |
| | BRENNER TUMOR | | 1 |
| | CARCINOSARCOMA | | 2 |
| | CLEAR CELLS | | 6 |
| | ENDOMETRIOID | | 6 |
| | MUCINOUS | | 6 |
| | NA | | 34 |
| | SEROUS | | 59 |
| tumor/metastasis | METASTASIS | | 33 |

(continued)

|  | Value | No. of Patients |
|---|---|---|
| Breast tumor_Age at diagnosis | PRIMARY Median=48 years | 83 |
|  |  | 103 |
|  | 20-39 | 2 |
|  | 40-49 | 7 |
|  | 50-59 | 2 |
|  | 60-69 | 2 |
|  | 70-79 | 0 |
|  | 80-89 | 0 |
| Breast tumor_Local treatment | BCS | 5 |
|  | M | 8 |
|  | NA | 103 |
| Breast tumor_Stage | I | 2 |
|  |  | 1 |
|  | III | 10 |
|  | NA | 103 |
| Breast tumor_Systemic treatment | CT | 5 |
|  |  | 5 |
|  | NA | 103 |
|  | NO | 1 |
|  | T | 2 |
| Breast_ER | plus | 10 |
|  | minus | 3 |
|  | NA | 103 |
| Breast_ Grade | I | 2 |
|  | II | 3 |
|  | III | 7 |
|  | III, THEN II | 1 |
|  | NA | 103 |
| Breast_Histology | IDC | 6 |
|  | AND ILC (MIXED) | 1 |
|  | IDC, THEN ILC* | 1 |
|  | ILC | 4 |
|  | NA | 103 |
|  | UNDIFFERENTIATED | 1 |
| Breast_PgR | positive | 9 |
|  | negative | 4 |
|  | NA | 103 |
| Ovarian tumor_Age at diagnosis | Median | 52 |
|  | NA | 103 |
|  | 20-39 | 1 |
|  | 40-49 | 5 |
|  | 50-59 | 5 |
|  | 60-69 | 1 |
|  | 70-79 | 1 |
|  | 80-89 | 0 |
| Ovarian tumor_FIGO stage | IC | 1 |
|  | IIA | 2 |
|  | IIC | 1 |
|  | IIIC | 4 |

(continued)

|  | Value | No. of Patients |
|---|---|---|
|  | IV | 5 |
|  | NA | 103 |
| Ovarian tumor_Metastatic site | BONE, LIVER | 1 |
|  | LIVER | 2 |
|  | NA | 111 |
|  | PERITONEAL CARCINOMATOSIS, SUPRA | 1 |
|  | PLEURA | 1 |
| Ovarian tumor_Outcome | DOD | 9 |
|  | NA | 103 |
|  | NED | 3 |
|  | SD | 1 |
| Ovarian tumor_Time interval from BC (months) | Median=34 |  |
|  | NA | 103 |
| Ovary_ER | positive | 4 |
|  | negative | 2 |
|  | NA | 103 |
|  | ND | 7 |
| Ovary_grade | II | 2 |
|  | III | 4 |
|  | NA | 103 |
|  | ND | 7 |
| Ovary_Histology | LOBULAR CARCINOMA | 5 |
|  | NA | 103 |
|  | POORLY DIFFERENTIATED ADK | 3 |
|  | SEROUS PAPILLARY | 5 |
| Ovary_Pgr | positive | 3 |
|  | negative | 1 |
|  | NA | 103 |
|  | ND | 9 |

Normalization of the expression values of each dataset was performed with MBEI algorithm (Li C., *et al.*, 2001). ANOVA method (Kerr M.K. *et al.,* 2001) was utilized to adjust the batch effect among three datasets. All procedures described for TCGA expression data analysis were performed in a similar manner in the GEO expression datasets.

EXAMPLE 1

**EVI1 gene expression is a discriminative marker between normal and cancer ovarian tissues**

The results are shown in Figure 1 where the expression of EVI1 and MDS1 genes of MECOM locus were shown to be strongly increased in ovarian cancer cells, in comparison with normal epithelium. In the samples taken from ovarian tissues using laser microdissection technique, the average expression of EVI1 and MDS1 genes were 3.6 and 1.4 times higher respectively in cancer tissues, in comparison with neighboring normal tissues (Figure 1). This difference in the mean expression value is more significant for EVI1, in comparison with MDS1 ($P$=0.021). MDS1 and EVI1 expression values correlate across all the samples (Pearson's r=0.84, Kendall's $\tau$=0.60).

**EXAMPLE 2EVI1 expression was a sensitive and specific marker of primary EOC tumors among conventional diagnostic biomarkers**

To assess the significance of EVI1 in ovarian cancer, its expression was analyzed in three data sets and its significance as a diagnostic marker was tested against a set of genes, which significance in diagnostics of ovarian cancer and patients survival is widely accepted: KRAS, ERBB2, P53, MYC, MUC16 (CA-125) and WFDC2 (HE4). The results against WFDC2 and MUC16 are shown in Figure 2. As can be seen from the results, MECOM expression discriminates between normal and cancerous ovarian epithelia. The combination of expression values of MECOM genes alone Figure 2(A) discriminated EOC with False negatives (FN) =1 and False positives (FP) =1, which was comparable to the combination of MUC16 (CA-125) and WFDC2 (HE-4) markers Figure 2(C). The perfect separation was obtained in the combination of EVI1 and MUC16 genes Figure 2(B). The overall discriminative power of EVI1 gene for normal vs. EOC epithelia was comparable with MUC16 and WFDC2 Figure 2(D).

The expression values of each marker in ovarian tumors emerged from ovarian epithelia (primary tumors) were compared with with the corresponding expression values in the ovarian metastases of breast cancer tumors (secondary tumors). In the given arrays the expression of EVI1 was represented by probesets 226420_at for MDS1 (RefSeq MECOM, transcript variant 4, NM_004991.3; SEQ ID NO:4) and 208434_at for EVI1 (RefSeq MECOM, transcript variant 2, NM_005241.3; SEQ ID NO:6) transcripts respectively. The expression cutoff value (given in a log2 scale) was selected in such way that it minimized the number of false negative diagnoses.

The results presented on Figures 3 and 4 demonstrate that both MDS1 and EVI1 genes can discriminate between primary and secondary ovarian tumors with the highest specificity ($P = 6.10^{-14}$ and $P = 6.10^{-12}$ respectively) among the conventional markers (P53, KRAS, ERBB2, MYC, WFDC2 and MUC16).

Cumulative distribution curves of gene expression values presented in Figure 3 to discriminate A) between primary EOC and breast cancer metastases in the ovaries; B) between low malignancy potential tumors and malignant EOC. The larger the difference between the grey and the black cumulative curves observed (by the P-value), the larger the diagnostic value of the given gene. As can be seen in Figure 3, EVI1 and MDS1 genes of MECOM locus were more sensitive and specific in EOC diagnostics than many conventional biomarkers.

Figure 4 shows that both EVI1 and MDS1 genes of MECOM locus are important for diagnostics. Discrimination between Low malignant potential (LMP) and malignant primary EOC as shown in Figure 4 improved after combining the expression values of EVI1 and MDS1 genes together in a two-dimensional discriminant function.

At expression cutoff level 8.5 of EVI1 expression the number of false negative (FN) diagnoses of primary EOC was $FN_{EVI1}=7$ with the number of false positive (FP) $FP_{EVI1}=0$. For the next powerful biomarker MUC16 (CA-125) at expression cutoff level 6.7 $FN_{MUC16}=8$ and $FP_{MUC16}=5$. Thus, for EVI1 gene the specificity = 33/(33+0)=100% and the sensitivity = (83-7)/83=91%, while for MUC16 the specificity = 33/(33+5)=87% and the sensitivity = (83-5)/83=94%. The robustness of the discrimination between primary and secondary ovarian tumors can be improved by measurement of both EVI1 and MDS1 genes simultaneusly as shown in Figure 4.

As can be seen, the transcripts of genes of MECOM locus are more sensitive and specific in EOC diagnostics than many conventional biomarkers. The Receiver Operating Characteristic (ROC) curves of Figure 5 showed the expression of EVI1 gene of MECOM locus as a biomarker for clinical diagnostics in comparison with two currently most successful diagnostic markers WFDC2 (HE-4) and MUC16 (CA-125). The best predictor was defined by the curve closest to the coordinate axes. The worst predictor was defined by the curve closest to the reference line (false positive rate = true positive rate).

The specificity and the sensitivity of the discrimination between primary and secondary ovarian tumors is further improved by measurement of EVI1 together with transcripts of ERBB family genes and their secreted signalling protein ligands as shown in Figure 6.

The secreted ligands (such as EGF, TGFA, NGR1) demonstrated slightly lower sensitivity, in comparison with ERBB family receptors (such as ERBB1, ERBB2, ERBB3) as shown in Figure 6.

For EVI1-NGR1 combination the sensitivity was (83-4)/83=95% and the specificity was 100%.

For EVI1-EGF and EVI1-TGFA combinations the sensitivity was (83-3)/83=98% and the specificity was 33/(33+1) = 97%.

For EVI1-ERBB1 and EVI1-ERBB2 combinations the sensitivity was (83-2)/83=98% and the specificity was 100%.

For EVI1-ERBB3 combination the sensitivity was (83-1)/83=99% and the specificity was 100%.


**EXAMPLE 3**


**EVI1 expression was a sensitive marker of EOC tumor malignancy potential**


In respect to the malignancy potential of the tumors EVI1 gene was ranked 5th (P=0.022), surpassing biomarkers MYC (*P*=0.03), WFDC2 (*P*=0.32) and ERBB2 (*P*=0.74) in the group discrimination significance.

For EVI1 at expression level cutoff 9.5 $FN_{ENVI1}=18$, $FP_{ENVI1}=4$, the specificity = 21/(21+4)=84% and the sensitivity = 49/(49+18)=73%, while for MUC16 (CA-125) at expression cutoff level 9.7 $FN_{MUC16}=11$, $FP_{MUC16}=5$, the specificity = 21/(21+5)=81% and the sensitivity = 49/(49+11)=82% The advantage of EVI1 as a clinical diagnostic biomarker proposed in the current innovation over the two certified clinical biomarkers MUC16(CA-15) and WFDC2(HE4) in the full range of cutoff values separating the patient groups is presented on the comparative plots of ROC curves on Figure 6. Discrimination between primary EOC tumors and breast cancer metastases in the ovaries was improved after combining the expression values of EVI1 and ERBB1 genes together in a two-dimensional discriminant function as shown in Figure 6. A combination of EVI1 and ERBB1 expression was shown to be the most sensitve marker of primary EOC.

## EXAMPLE 4

**The prognostic significance of MDS1 gene expression depends on the number of its genomic copies in tumor tissues, while EVI1 expression is survival significant regardless of its gene copy number**

The prognostic significance of the expression of EVI1 and, especially, MDS1 was strongly dependent of the copy numbers of their genomic regions in the patients. Survival analysis of the patients with EOC diagnosed at stages 3 and 4 demonstrated that EVI1 is one of the three genes, which expression level separates the patients into two groups with significantly different survival times. As a survival prognostic marker, the expression of EVI1 gene with $P=0.010$ ranked second after PIK3CA ($P=0.0021$). The survival significance of MDS1 gene expression with $P=0.049$ was marginal, ranking behind PIK3CA, EVI1, WFDC2, MUC16, KRAS, and MYC, but better than ERBB2, P53 and BRCA2.
In the studied population EVI1 gene was represented in more than 2 copies per genome in 52% (262/504) of the patients. The values of both average copy number and the fraction of the patients exceed the corresponding values (50 to 70%) reported previously for EOC tumors (Sunde JS, 2006). The site of copy number at the 3' end of MECOM locus was the most highly amplified region on chromosome 3 reaching the strongest gene amplification hot spot in the genomes of EOC tumor cells as shown in Figure 7. The 3' end of the MECOM locus copy number was 2.5 and higher in the tumors of 84% of patients with EOC. Across the patients, EVI1 region was characterized with mean 3.52 and median 3.19 copies per genome, and MDS1 region with mean 3.39 and 3.18 copies respectively as shown in Figure 8.
The effect of EVI1 and MDS1 expression was analysed in the tumors on the survival of patients as factors depending on the genomic amplification of these genes. The effect of EVI1 and MDS1 copy numbers was studied by separating the population of patients into two groups relative to a given copy number cutoff: the patients with a large number of EVI1 or MDS1 copies (the genes are amplified) in their tumors and the rest of the patients (the genes are not amplified). The results are presented on Figure 8C and D. Survival analysis of EVI1 and MDS1 expression was performed in the high copy number group and the best P-values for the survival groups separated by the expression were calculated. The copy number cutoff value varied from 0 to 4. Thus copy number-dependent expression-defined survival P-value was calculated. Figure 8(A) shows the comparison of the best separation of the patient survival curves by gene expression markers (grey- high expression, black-low expression); the more separated grey and black curves were (the less the p-value), the more predictive power for patient survival a given marker had. Histograms in Figure 8(B) are of amplification values of EVI1 and MDS1 genes of MECOM locus obtained. The dependence of the p-value of the best survival curves separation (Pmin) on the degree of amplification of the genes from MECOM locus obtained are shown in Figure 8(C). Figure 8(D) are survival curves which show the maximal separation of patients for the subsets of patients with EVI1 and MDS1 obtained. The amplification higher (the upper row) and lower (the middle row) than 3.5 and 3.6 copies/cell were obtained respectively. As a reference of survival curves separation the combined (amplified together with not amplified) are given in the bottom row. In Figures 8(A) and (D) the cutoff expression value (in log2 scale) are denoted with letters above each of the graphs (within the graph frames). For Figure 8A and the 'Combined' graphs in Figure 8(D) the cutoff was obtained with a single one-dimensional optimization by the expression value. In Figure 8(D) the analysis was made with a two-step optimization by 1) copy number, followed by 2) expression. It is shown that amplification of MECOM locus together with the expression of its genes EVI1 and MDS1 were stronger indicators of EOC patients' survival prognosis than many conventional biomarkers.
The copy number cutoff giving the best survival P-value for EVI1 and MDS1 was slightly different (3.5 and 3.6 respectively), although these loci are adjacent. In the group of patients, where MDS1 copy number was higher than 3.6, the expression of MDS1 was a strong factor separating the patients by survival ($P=0.0028$), showing a remarkable improvement of the P-value by a factor of 17, in comparison with the whole population ($P=0.049$). In the sub-population where MDS1 was not amplified, no survival significance of its expression was observed ($P=0.39$).
MDS1 expression was a significant factor of EOC patients' survival only if in their tumors MDS1 gene region copy number was higher than 3.6 (227/354, 65% of the patient population). At the same time, EVI1 gene expression was a significant factor of patients' survival for the whole population ($P=0.010$), regardless of the level of the amplification of its coding region. The results are shown on Figure 8D.
The survival prognostic value of both EVI1 and MDS1 gene depended on their copy number (i.e. the copy number of MECOM locus, which includes both of them). As displayed on Figure 8D, after segregation of the patients ("Combined") into 2 cohorts ("Amplified" and "Not amplified") by the genes copy number, the prognostic P-value for EVI1 was improved. In addition, for EVI1 gene the prognostic value was marginally significant even without prior patient stratification ("Combined" cohort) by copy number values. Thus, EVI1 gene expression can be used as a prognostic marker regardless its copy number status.

The results demonstrated that MECOM locus, including proto-oncogenes EVI1 and MDS1, was a clinical biomarker for differential diagnostics and prognosis of the human epithelial ovarian cancer.

## EXAMPLE 5

### EVI1 and MDS1 were strong prognostic factors for shorter survival periods

The P-value of the difference between MECOM copy number distributions for patients with good (survival) and poor (death) prognoses for a given limited time period had a log-linear dependence on the time limit with a strong positive correlation as shown in Figure 14.

Detailed correlation structure of surviving (good prognosis) and deceasing (poor prognosis) patient cohorts were defined by follow-up time. Both MDS1 and EVI1 expression P-value (between good and poor prognosis cohorts) correlated with patients' follow-up time. For MDS1 two ranges of follow-up times with different correlation coefficients were observed.

For both EVI1 and MDS1 the functional dependence of P-value on the survival time limit consists of two trends with very strong correlation separated by a region of discontinuity around time 1200 days. For time periods shorter than 500 days correlation between P-value and copy number decreases, while MECOM copy number became a stronger marker, in comparison with all conventional cancer biomarkers as can be seen when comparing Figures 9 and 10. As can be seen in Figure 9, expression of EVI1, but not MDS1 gene showed a strong survival significance for the given prognosis time span. Based on Figure 9, EVI1 was shown to be the most powerful marker for discriminating between poor and good prognosis patients at a short prognostic time period (300 days). As can be seen in Figure 10, expression only of either EVI1 or MDS1 did not have a sufficient survival prognostic power for the given prognosis time span. However, the expression of both genes was highly significant for patients (91%) with tumors with amplified MECOM locus (copy number >2.9). EVI1 was thus shown to be a marginally significant marker for discriminating between poor and good prognosis patients at medium prognostic time period (1500 days).

## EXAMPLE 6

### The genes of MECOM locus (EVI1 and MDS), being analyzed simultaneously, improve the discriminative power of clinical analysis

The combination of expression data of MDS1 with EVI1 could be used together for increasing the discrimination power of ovarian tumor subtypes in. This possibility is illustrated on Figure 3B by a comparison of the cumulative frequency distribution of the genes expressed in low malignancy potential (LMP) tumors versus malignant EOC. On this figure it can be observed that higher expression values of EVI1 gene of MECOM locus correspond to malignant EOC type. Contrary to this, higher expression values of MDS1 gene of this locus correspond to LMP ovarian tumor type. Thus, the maximal instrumental sensitivity (with both genes measured at high expression) of the measurement is obtained. If MDS1 and EVI1 expression was measured together, the power of the resulting two-dimensional discriminant function to separate the LMP and malignant EOC subgroups was increased, as shown on Figure 4.

## EXAMPLE 7

### EVI1 and MDS1 copy number were predictive markers of primary therapy outcome

The P-value was studied for the difference between EVI1 and MDS1 copy number distributions for patient groups with good prognosis and poor prognosis. For a given follow-up time cutoff the poor prognosis group was limited to the patients who died before this follow-up time. The good prognosis group was limited to the patients who survived longer than the given follow-up time.

These P-values were found to be different for patients grouped by primary therapy outcome success ("complete", "incomplete" and "null" response patient cohorts), as seen, e.g. on Figures 9 and 10. Moreover, the P-values for both EVI1 and MDS1 strongly correlated with the fraction of patients belonging to each cohort in the poor prognosis group as shown in Figure 11 which shows that the primary therapy response outcome depends on EVI1 and MDS1 copy numbers.

The correlation structure for each cohort was complex. For EVI1, with increase of the cohort patient fraction up to a certain limiting value (10% for "complete", 40% for "incomplete" and 50% for "null" response groups for EVI1) the correlation with P-value was strongly negative (-0.58 for "complete", -0.6 for "incomplete" and -0.89 for "null" response groups for EVI1). After this limit, the correlation changed to strongly positive for "complete" and "null" response groups, but remained negative for "incomplete" response group. For MDS1 the correlation structure was qualitatively similar. Thus, an 'optimal' fraction of patients for a certain therapy response was defined by the expected minimal P-value of EVI1 (or MDS1) copy number distribution different between the group of the patients with good and poor survival prognosis.

This demonstrated a dependence of primary therapy outcome response on the copy number of EVI1 and/or MDS1 in the tumor tissue of a given patient, along with survival prognostic time period. It became evident that every distribution of EVI1 and MDS1 values predicted the 'optimal' fraction of patients with a given time of response. This optimal corresponded to the maximal dependence of therapy response outcome (as survival probability) of patients in a given cohort on the expression of EVI and/or MDS1.

The expression value of EVI1 and/or MDS1, measured at the time of the surgery, simultaneously mapped each individual patient to the probability distribution of the difference between good and poor survival groups, patient survival probability and the probability of having a "complete", "incomplete" and "null" response and the expected survival time. Thus for a given patient primary therapy outcome could be predicted, along with the survival time estimated from this prediction. An example of this analysis is shown on Figure 17 which includes the algorithm for primary therapy outcome prediction and associated patient survival prognosis based on EVI1/MDS1 copy number data.

The specific steps include:

1. Measure EVI1/MDS1 copy number in the tumor extracted during surgery. Map the copy number on the copy number distribution of EVI1/MDS1.
2. For the given copy number find its quantile Q in the copy number distribution. Optional: for a given copy number find its quantile in the copy number distribution in each response cohort ("Complete", "Incomplete" and "Null") separately . These alternative quantiles will lead to 3 alternative prediction scenarios, the results of which at Step 7 can be combined.
3. Map Q to the "response cohort class composition" vs. Q (copy number) nomogram (Figure 11)
4. From the points of intersection of line "$y=Q$" with the approximated cohort fraction - log(Q) dependence the corresponding values on Ox axis are estimated for each cohort. Thus the inference of the plausibilities of the given patient belonging to each of the cohorts (Ox axis) is made. Up to 6 potential expected cohort fraction values (6 plausibility values) can be obtained at this step if all the intersection points are within [0,1] range. Similarly to Step 2, these values can be traced further to be resolved at Step 7.
5. Map the obtained set of expected cohort fraction values (plausibilities) to "Last follow-up time" vs. "cohort fraction" nomogram (Figure 15A).
6. For every given expected cohort fraction (plausibility) find the points of intersection of the corresponding lines "" with the nomogram curves for each corresponding primary therapy outcome prediction cohort. Obtain the corresponding values of expected survival times on Ox axis for each cohort fraction.
7. Obtain the predicted survival time based on the primary therapy outcome predictions by inferring it from the plausible survival times obtained at Step 6. For example, at Step 2, 6 points of intersection (2 points for each of the 3 quantiles were obtained). At Steps 5 and 6 each point was mapped to a single expected survival time. Thus 6 survival times was obtained. If the goal is to obtain the prediction at the time of obtaining copy number data (i.e. at the time of surgery), a range between the minimal and the maximal of the 6 survival times can be retrieved as a prediction.
8. The set of plausible survival times (up to 6) can be reduced to the set of 2 survival times if the survival prognosis is made for the patient after the outcome of the primary therapy is observed. Then, the certainty in the patient primary therapy outcome cohort is obtained and thus the range of survival times is reduced to the smallest and the largest predicted values for the given cohort.

Primary therapy response strongly correlated with patient last follow up time Figure 15(A), which, in its turn, correlates with EVI1 Figure 15(B) and MDS1 Figure 15(C) -log10 of P-values for expression differentiating between patients with good and poor prognosis. Good and poor prognosis cohorts for a given follow-up time are defined by patient survival or death by the given follow-up time.

## EXAMPLE 8

### EVI1 and MDS1 were predictive markers for secondary therapy outcome

For a short-term survival period (600 days) survival, patients who did not receive secondary chemotherapy, died significantly more often (EVI1 P=0.01, MDS1 P=0.003) if their tumors contained high copy number of MECOM. The 12secondary therapy outcome prediction results is shown in Figure 12. Among the patients who received secondary chemotherapy, MECOM copy number was an insignificant factor of survival. Figure 12(A) show the results of patients who received chemotherapy and Figure 12(B) show the results of patients who did not receive chemotherapy.

EVI1 expression was insignificantly different among the patients, regardless of their receiving chemotherapy.

However, MDS1 expression was marginally higher (P=0.04) in the group of patients with poor prognosis who received secondary chemotherapy.

## EXAMPLE 9

**EVI1 and MDS1 expression and copy number were strong long-term prognostic factors in each group of patients separated by response type to primary therapy**

Combinations of EVI1 and MDS1 copy number and expression values separate groups of patients with various types of response to primary therapy. These markers can be also considered as factors increasing the prognostic power of primary therapy outcomes13. A schema for a robust survival prognosis using a combination of MECOM copy number and EVI1 and MDS1 expression with primary therapy response outcome is shown in Figure 13. For patients with complete response and MECOM copy number between 2.8 and 4.2, EVI1 expression less than 10.3 and MDS1 expression less than 6.76 long survival time was predicted, while for patients with EVI1 and MDS1 expression higher than these values medium survival time was predicted (up to 2000 days).

For patients with partial and no response (stable or progressive disease) to primary chemotherapy, if MECOM copy number was less than 2.7 and MDS1 expression was less than 6.43, medium survival time as predicted. For patients with MECOM copy number > 2.7 and EVI1 expression less than 9.51, medium survival time was predicted.

Short survival time (maximum 1000 days) was predicted for patients without complete response to primary chemotherapy, MECOM copy number <2.7 and MDS1 expression higher than 6.43, or, in the case of MECOM copy number >2.7, if EVI1 expression >9.51. Overall, lower MECOM copy number and EVI1 and MDS1 expression, as well as complete response in survival time resulted in prognosis of longer survival times.

## EXAMPLE 10

**The prognostic power of MECOM copy number was the strongest for patients with shortest surviving times and predicted for them negative outcome of primary therapy**

From Figure 15, the P-value of difference between MECOM copy number distributions for patients with good and poor prognoses correlated not only with the prognostic period, but also with primary therapy response group composition of the patients. As the fraction of patients with partial and complete response increased, both survival time and the copy number P-value increased. Contrary to this, the MECOM copy number (and EVI1 expression value) was the most significant survival-predicting factor for the short periods of time (<300 days, as shown on Figure 9), when most of the patients belonging to the "Null" therapy response group died. -log10 of P-values for expression differentiated between patients with good and poor prognosis. Good and poor prognosis cohorts for a given follow-up time were defined by patient survival or death by the given follow-up time.

Thus, regarding the prognostic value of MECOM copy number (and also EVI1 and MDS1 expression) and survival of patients grouped by primary therapy response, the prognosis time scale could be divided into 3 ranges (Figure 15A). Short term prognostic range, up to 500 days long, was characterized with the strongest prognostic power of MECOM copy number and the expression of its genes, as well as with the prevalence of the "Null"-therapy responding patients. Medium term prognostic range, spanning from 500 to 1500 days - was characterized with poor prognostic power of MECOM copy number and the expression of its genes (where some conventional cancer biomarkers show stronger prognostic power than MECOM), as well as prevalence of the mixed group of "Incomplete" primary therapy response (combining patients with partial response and stable or progressing disease). Long term prognostic range as characterized with MECOM copy number -dependent prognostic power of EVI1 and MDS1 expression (stronger than for conventional cancer biomarkers) and with prevalence of patients with complete response to primary therapy.

## EXAMPLE 11

**EVI1 and MDS1 were markers for anti-cancer therapy success prediction**

EVI1 expression value was a significant marker (P(Mann-Whitney U)=0.0021) for predicting the outcome of primary post-operative therapeutic treatment response at the time of surgery as shown in Figure 16A. MDS1 expression was insignificant with a trend to significance (P=0.074). In comparison with conventional cancer markers (e.g. P(MUC16)=0.7), P(WFDC2)=0.1, P(KRAS)=0.49, P(P53)=0.4, P(ERBB2)=0.37), only EVI1 expression demonstrated a statistically significant P-value and MDS1 expression ranked second after EVI1. EVI1 and MDS1 transcription was a significant prognostic factor in combination with patients with complete response to the primary therapy ("Complete response" group) are compared with a combined group containing patients demonstrating partial response, stable or progressive disease ("Incomplete response"). Figure 16(A) show the cumulative distribution functions of EVI1 and MDS1 gene expression values for "Complete response" (black dots) and "Incomplete response" (grey dots). Figure 16(B) show that EVI1 and MDS1 copy number (MECOM locus) and expression values as survival prognostic markers for patients with complete response to primary therapy. Figure 16(C) show EVI1 and MDS1 copy number (MECOM locus) and expression values as survival prognostic markers for patients with partial response to primary therapy, stable or progressive disease.

The prognostic value of EVI1 and MDS1 for primary therapy response was further confirmed by studying patients segregated by primary response types. Both genes were significant for survival prognosis in both patient groups (i.e. complete and "incomplete" response) for. MDS1 was a significant prognostic marker for 42% of patients with complete response (P=0.0010) and 64% of patients with "incomplete" response (P=0.0045). EVI1 was a significant prognostic marker for 82% of patients with complete response(P=0.0048) and 50% of patients with "incomplete" response (P=0.018).

An illustration of primary therapy success prediction is given on Figure 13. On the first stage the prediction of primary therapy success is made based on initial data on MECOM copy number and MDS1 and EVI1 expression values obtained from the analysis of tumor sample extracted during the surgery. Based on this data an appropriate course of therapy is selected (more intensive in the case of MECOM amplification and less intensive otherwise). On the second stage, the response to primary therapy is assessed. Based on the type of response further decision is made on the more exact prognosis of survival time of the patient based on the exact range of MECOM copy number and MDS1 and EVI1 expression values.

## EXAMPLE 12

### EVI1 expression is decreased after treatment with neo-adjuvant chemotherapy

Nine samples of malignant epithelial ovarian cancer tumors were compared with 24 samples of epithelial ovarian cancer tumors obtained from patients who received neo-adjuvant chemotherapy prior to the surgerical treatment (13. Moreno CS, 2007). Expression of EVI1 was measured with probesets 1881_g_at and 1882_g_at probesets of Affymetrix HG-U95Av2 microarray. Both probesets demonstrated a significant difference (P=0.0032 and P=0.032 respectively) in expression level of EVI1 between the two groups. The data is presented in Figure 18. In the same cohort of patients it was also observed that EVI1 expression was different in benign (adenoma) compared to malignant ovarian tumors (Figure 19). In the latter case, the difference was more significant (P=0.0014 and P=0.007 respectively) and suggests that treatment inefficiency and high malignancy are associated with high expression of EVI1.

## EXAMPLE 13

### EVI1 expression is a potenital diagnostic marker for a range of genitourological tumors.

EVI1 expression was found to be altered in some other gynecological tumors. In cervical cancer (Scotto L, 2008] EVI1 expression profile qualitatively resembled the one in EOC and EVI1 level was significantly elevated ((P=1.1E-6) in cervical cancer tumors compared to normal cervical epithelium (Figure 20). Contrary to this, in endometriosis tumors (Hever A, 2007) EVI1 transcript expression was not only significantly lower (P=0.00016) than in normal endometrium, but also was a discriminative (100% specificity and sensitivity) characteristic of this tumor (Figure 21). In this test MDS1 transcript revealed a similar significance (P=0.00021) and a comparable discriminative power (100% sensitivity, 90% specificity).

Similarly, in clear cell renal cell carcinomas (Gumz ML, 2007) high EVI1 expression was associated with normal epithelium (P=0.00016) with 100% specificity and sensitivity (Figure 22).

## EXAMPLE 14

### Primers proposed in the present invention to measure EVI1 expression are specific and sensitive biomarkers for both diagnostics and survival prognosis of ovarian cancer.

primer sequences suitable for PCR-based techniques (including, but not limited to qPCR) and the sequence for a fluorescent probe, which can specifically measure the expression level of EVI1were designed. A forward (EVI1-For) sequence 5'-GGTTCCTTGCAGCATGCAAGACC-3' (SEQ ID NO:1) and reverse primer (EVI1-Rev) sequence 5'-GTTCTCTGATCAGGCAGTTGG-3' (SEQ ID NO:2) along with fluorescent probe (EVI1-Flu) FAM6-TACTTGAGGGCTTCTCCAGG-TAMRA (SEQ ID NO:3), were designed targeting EVI1 isoform. The EVI1 isoform was selected from the group consisting of SEQ ID NO:4, 5 and 11-15. Primers were designed for endogenous control beta actin (Forward-CAGCCATGTACGTTGCTATCCAGG (SEQ ID NO:7), Reverse-AGGTCCAGACGCAGGATGGCATG (SEQ ID NO:8) and fluorescent probe- FAM-actggcatcgtgatggactc-TAMRA SEQ ID NO:9)) for relative quantification studies. Each primer concentration was optimized and subsequently used on tissue array qPCR panel I and II for gene expression studies. PCR reaction was run on 7500 ABI machine using Taqman universal master mix (cat.no: 4304437) and CT values were obtained and relative quantification was estimated using ddCT method (Livak KJ, 2001).

The obtained fold change values were used for the analysis. Two panels of actin normalized commercial ovarian cancer tissue array qPCR plates (96 wells) HORT01, HORT02 (Origene technologies) were used for primer validation. Each panel contains 48 patient samples of cDNA (normalized with actin) that included normal and stage specific ovarian cancer patients for relative comparison of gene expression of various potential biomarkers. Both panels had unique patient ID's and a total of 15 normal and 81 ovarian patient sample cDNA with various stages enlisted as follows: stage IA=11, stage IB=6, stage IC=7, stage IIA=3, stage IIB=6, stage IIC=3, stage IIIA=11, stage IIIB=11, stage IIIC=14, stage IV=9. Primer3 open source software was used to design forward and reverse primers along with fluorescent probes for qPCR studies.

Fold change distribution analysis (Figure 23) demonstrated that the primers, measuring EVI1 expression, with 100% specificity and sensitivity discriminate between normal ovarian surface epithelium and ovarian cancer tumors at any stage (P-values ranging from 6E-13 to 2E-6 on Figure 23B) and can be used to determine favourable and ufavourable survival prognoses of the patients (P=0.011) by the optimal EVI1 expression cutoff (Fold change to normal ovarian epithelium 16.76 on Figure 23C).

**REFERENCES**

1. Anglesio MS, Arnold JM, George J, Tinker AV, Tothill R, et al. (2008) Mutation of erbb2 provides a novel alternative mechanism for the ubiquitous activation of ras-mapk in ovarian serous low malignant potential tumors. Mol Cancer Res 6: 1678-90.

2. Bowen NJ, Walker LD, Matyunina LV, Logani S, Totten KA, et al. (2009) Gene expression profiling supports the hypothesis that human ovarian surface epithelia are multipotent and capable of serving as ovarian cancer initiating cells. BMC Med Genomics 2: 71

3. Edgar R (2002) Gene expression omnibus: Ncbi gene expression and hybridization array data repository. Nucleic Acids Research 30: 207.

4. Jazaeri AA, Ferriss JS, Bryant JL, Dalton MS, Dutta A (2010) Evaluation of evi1 and evi1s (delta324) as potential therapeutic targets in ovarian cancer. Gynecol Oncol 118: 189-95.

5. Kerr MK, Churchill GA (2001) Statistical design and the analysis of gene expression microarray data. Genet Res 77: 123-8.

6. Li C, Hung Wong W (2001) Model-based analysis of oligonucleotide arrays: model validation, design issues and standard error application. Genome Biology, 2(8):research0032.1-0032.11

7. McLendon RE, Friedman AH, D BD (2008) Comprehensive genomic characterization defines human glioblastoma genes and core pathways. Nature 455: 1061-8.

8. Meyniel JP, Cottu PH, Decraene C, Stern MH, Couturier J, et al. (2010) A genomic and transcriptomic approach for a differential diagnosis between primary and secondary ovarian carcinomas in patients with a previous history of breast cancer. BMC Cancer 10: 222

9. Sambrook and Russel, Molecular Cloning: A Laboratory Manual, Cold Springs Harbor Laboratory, New York (2001).

10. Sunde JS, Donninger H, Wu K, Johnson ME, Pestell RG, et al. (2006) Expression profiling identifies altered expression of genes that contribute to the inhibition of transforming growth factor-beta signaling in ovarian cancer. Cancer Res 66: 8404-12

11. http://www.ncbi.nlm.nih.gov/UniGene/clust.cgi?ORG=Hs&CID=659873

12. Nanjundan M, Nakayama Y, Cheng KW, Lahad J, Liu J, Lu K, Kuo WL, Smith-McCune K, Fishman D. Gray JW and Mills GB. Amplification of MDS1/EVI1 and EVI1, Located in the 3q26.2 Amplicon, Is Associated with Favorable Patient Prognosis in Ovarian Cancer. Cancer Res, 2007 67:3074-3084

13. Moreno CS, Matyunina L, Dickerson EB, Schubert N, Bowen NJ, Logani S, Benigno BB and McDonald JF. Evidence that p53-mediated cell-cycle-arrest inhibits chemotherapeutic treatment of ovarian carcinomas. PLoS One. 2007 2:e441

14. Scotto L, Narayan G, Nandula SV, Arias-Pulido H, Subramaniyam S, Schneider A, Kaufmann AM, Wright JD, Pothuri B, Mansukhani M and Murty VV. Identification of copy number gain and overexpressed genes on chromosome arm 20q by an integrative genomic approach in cervical cancer: potential role in progression. Genes Chromosomes Cancer. 2008 47:755-65

15. Hever A, Roth RB, Hevezi P, Marin ME, Acosta JA, Acosta H, Rojas J, Herrera R, Grigoriadis D, White E, Conlon PJ, Maki RA and Zlotnik A. Human endometriosis is associated with plasma cells and overexpression of B lymphocyte stimulator. Proc Natl Acad Sci U S A. 2007 104:12451-6

16. Gumz ML, Zou H, Kreinest PA, Childs AC, Belmonte LS, LeGrand SN, Wu KJ, Luxon BA, Sinha M, Parker AS, Sun LZ, Ahlquist DA, Wood CG and Copland JA. Secreted frizzled-related protein 1 loss contributes to tumor phenotype of clear cell renal cell carcinoma. Clin Cancer Res. 2007 13:4740-9

17. Livak KJ and Schmittgen TD. Analysis of relative gene expression data using real-time quantitative PCR and the 2(-Delta Delta C(T)) Method. Methods. 2001 25:402-8

18. Ferris: "Poster Session", Gynecologic Oncology, vol. 108, no. 3, 1 March 2008

19. Brooks D J et al. "Expression of the Zine Finger Gene EVI-1 in Ovarian and Other Cancers", British J. of Cancer, vol 74, no. 10, 1996

20. Johnson et al. "Identification of EVI1 amplification in ovarian cancer by oligonucleotide array comparative genomic hybridization". Can. Res. 2007

21. Morishita K et al. "Unique expression of the human Evi-1gene in an endometrial carcinoma cell line: sequences of cDNAs and structure of alternatively spliced transcripts." Database Medline. US National Library of Medicine (NLM) Bethesda, MD, US. Database acc. O. NLM2115646 & Oncogene 1990

22. Nanjundan M. et al. "Amplification of MDS1/EVI1 and EVI1 located in the 3q26.2 amplicon, is associated with favorable patient prognosis in ovarian cancer. Can. Res. Vol 67. 2007.

## Claims

1. An *in vitro* method for obtaining information in relation to a medical condition in a subject, the method comprising determining in a sample of the subject:

   (i) gene expression level of at least one gene in the MECOM locus, selected from *EVI1* and *MOS1;* and/or
   (ii) copy number of at least one gene in the MECOM locus selected from *EVI1* and *MOS1*;

   wherein the information is selected from the group consisting of:

   a) whether the subject has epithelial ovarian cancer, preferably high grade serous ovarian carcinoma and/or a predisposition to epithelial ovarian cancer, preferably high grade serous ovarian carcinoma, wherein the gene expression level against at least one statistically optimized gene expression cutoff value derived from a plurality of training subjects with known diagnosis and/or the at least one gene copy number against at least one statistically optimized gene copy number cutoff value derived from a plurality of training subjects with known diagnosis is/are indicative of said information, wherein said optimized cutoff value is obtained with the following steps;

      (i) extract the expression level or copy number level of a gene for each training subject;
      (ii) obtain the cumulative distribution function of the gene expression or copy number levels of two sets of training subjects, one set being associated with a positive diagnosis and one set being associated with a negative diagnosis; and
      (iii) select a cut-off value which divides the training subjects into two groups, based on whether the gene expression level or copy number level of each training subject is higher or lower than the cut-off value, and which minimizes the false positive and/or false negative rate in the diagnosis using one-dimensional statistical optimization;
      or, if the expression and the copy number values are identified in the same sample, the optimal cutoff for both expression and copy number are obtained simultaneously by minimising the false positive and/or false negative rates using two-dimensional statistical optimization;

   wherein said epithelial ovarian cancer determination comprises using PCR primers comprising a forward primer SEQ ID NO: 1 and reverse primer SEQ ID NO: 2 and probe SEQ ID NO: 3, or using at least one of the probe sets selected from 1881_g_at, 1882_g_at, 208434_at, 221884_at and 226420_at;
   b) survival prognosis of the subject when the subject has epithelial ovarian cancer, preferably high grade serous ovarian carcinoma, wherein the expression level against at least one statistically optimized gene expression cutoff value and/or the at least one gene copy number against at least one statistically optimized gene copy number cutoff value is/are indicative of said information, wherein, for primary patient stratification, the optimal copy number and expression cutoff values are obtained for each gene using a two-step optimization algorithm as follows;

(i) extract the expression level and copy number of the gene for each training subject;

(ii) statistically estimate a copy number cutoff value and divide the training subjects into two cohorts according to whether the copy number of the gene is above or below the estimated copy number cutoff value; and

(iii) for each said cohort, select a gene expression cutoff value that divides the training subjects in each cohort into two groups based on whether the gene expression level of each training subject is higher or lower than the expression cutoff value, wherein the cutoff value minimizes the Wald test P-value of Kaplan-Meier survival models;

wherein said survival prediction determination comprises using PCR primers comprising a forward primer SEQ ID NO: 1 and reverse primer SEQ ID NO: 2 and probe SEQ ID NO: 3, or using at least one of the probe sets selected from 1881_g_at, 1882_g_at, 221884_at and 226420_at;

c) effectiveness of treatment of epithelial ovarian cancer, preferably high grade serous ovarian carcinoma, in the subject, wherein the gene expression level against at least one statistically optimized gene expression cutoff value and/or the at least one gene copy number against at least one optimized gene copy number cutoff value are indicative of said information;

wherein said effectiveness of treatment of epithelial ovarian cancer determination comprises using PCR primers comprising a forward primer SEQ ID NO: 1 and reverse primer SEQ ID NO: 2 and probe SEQ ID NO: 3, or using at least one of the probe sets selected from 1881_g_at, 1882_g_at, 221884_at and 226420_at;

d) whether epithelial ovarian cancer, preferably high grade serous ovarian carcinoma, in the subject is of primary or secondary origin, wherein the gene expression level against at least one statistically optimized gene expression cutoff value and/or the at least one gene copy number against at least one statistically optimized gene copy number cutoff value are indicative of said information;

wherein said determination of whether the epithelial ovarian cancer is of primary or secondary origin comprises using PCR primers comprising a forward primer SEQ ID NO: 1 and reverse primer SEQ ID NO: 2 and probe SEQ ID NO: 3, or using at least one of the probe sets selected from 226420_at, 221884_at and 208434_at; and

e) whether the subject has high grade serous ovarian carcinoma, cervical cancer, ovarian endometriosis, clear cell renal carcinoma and/or a predisposition to cervical cancer, ovarian endometriosis, clear cell renal carcinoma, wherein the level against at least one statistically optimized gene expression cutoff value and/or the at least one gene copy number against at least one statistically optimized gene copy number cutoff value are indicative of the subject having high grade serous ovarian carcinoma, cervical cancer, endometriosis, clear cell renal carcinoma and/or a predisposition to cervical cancer, endometriosis, or clear cell renal carcinoma;

wherein said determination of the type of cancer comprises using PCR primers comprising a forward primer SEQ ID NO: 1 and reverse primer SEQ ID NO: 2 and probe SEQ ID NO: 3, or using at least one of the probe sets selected from 226420_at, 1881_g_at, 1882_g_at, 221884_at and 208434_at,

wherein said gene expression level in a)-e) is determined from at least one transcript variant or isoform comprising a nucleic acid sequence selected from the group consisting of SEQ ID NO: 4, SEQ ID NO: 5, SEQ ID NO: 11, SEQ ID NO: 12, SEQ ID NO: 13, SEQ ID NO: 14 and SEQ ID NO: 15.

2. The method according to claim 1, wherein the information is in relation to epithelial ovarian cancer, preferably high grade serous ovarian carcinoma, which is adenocarcinoma or malignant ovarian cancer; and/or wherein the information is in relation to epithelial ovarian cancer, preferably high grade serous ovarian carcinoma, which is primary epithelial ovarian cancer.

3. The method according to any one of the preceding claims, wherein:

the gene expression level is determined by measuring the mRNA or protein expression of the gene from the MECOM locus; and/or

the method comprises determining the gene expression level of at least one further gene selected from the group consisting of MUC16, WFDC2, ERBB1, ERBB2, ERBB3, EGF, NGR1, and TGFA, in the sample.

4. The method according to any one of the preceding claims, wherein the training stage comprises the following steps for each said gene in the MECOM locus:

(i) for each of a plurality of training subjects with known diagnoses relating to ovarian cancer, preferably high grade serous ovarian carcinoma, determining gene expression level and copy number of the gene in the training subject;

(ii) estimating a sample copy number and dividing the training subjects into two cohorts according to whether the copy number of the gene in each training subject is above or below the sample copy number;

(iii) for each said cohort, determining a sample expression value which divides the training subjects in the cohort into two groups according to whether the gene expression level of the gene in each training subject exceeds the sample expression value, wherein the sample expression value achieves a maximum measure of difference between the two groups;

(iv) repeating steps (ii) - (iii) by varying the sample copy number in a range of copy numbers identified in the training subjects and obtaining a copy number distribution curve for the gene; and

(v) selecting a copy number cutoff value as the copy number associated with the largest measure of difference between the two groups of a cohort and selecting expression cutoff values as the expression values determined for the cohorts obtained with the copy number cutoff value,

the measure of difference between the two groups preferably comprising a measure of difference between survival curves of the two groups; wherein diagnosis of the subject, determining survival prognosis and/or determining effectiveness of treatment optionally further comprises the following steps for each said gene in the MECOM locus:

comparing the copy number of the gene of the subject against the copy number cutoff value for the gene; wherein

if the copy number of the gene of the subject exceeds the copy number cutoff value for the gene, further comparing the gene expression level of the gene of the subject against the expression cutoff value determined for the cohort with copy numbers of the gene above the copy number cutoff value;

if the copy number of the gene of the subject is below the copy number cutoff value for the gene, comparing the gene expression level of the gene of the subject against the expression cutoff value determined for the cohort with copy numbers of the gene below the copy number cutoff value; and

obtaining diagnosis of the subject, determining survival prognosis and/or determining effectiveness of treatment based on the comparison between the gene expression level of the gene of the subject and the respective expression cutoff value.

5. The method according to claim 4, for determining survival prognosis of the subject, wherein the method further comprises the steps of:

(i) parametrization of a dependence between a patient cohort fraction and the copy number of the gene in the set of training subjects;

(ii) parametrization of a dependence between the patient cohort fraction and the survival time in the set of training subjects, the survival time of the training subjects preferably being based on a last follow-up time for the training subjects; and

(iii) using the copy number of the subject to determine the patient cohort fraction from the dependence of (i) and using the patient cohort fraction of the subject to determine an estimated survival time of the subject from dependence (ii).

6. The method according to claim 4, for determining effectiveness of treatment, further comprising the steps of:

(i) parametrizing a dependence between the copy number of the gene of the training subject and a possible treatment response of the training subject selected from a group consisting of:

- a complete response;
- an incomplete response; and
- a null response; and

(ii) using the copy number of the subject to determine an estimated possible treatment response of the subject.

7. The method according to any one of the preceding claims, wherein the information is in relation to the epithelial ovarian cancer, preferably high grade serous ovarian carcinoma, and the method is capable of: determining whether the ovarian cancer in the subject is primary or secondary ovarian cancer; or whether the ovarian cancer has a malignancy potential in the subject.

8. The method according to any one of claims 1 to 6, wherein the information is the effectiveness of treatment of epithelial ovarian cancer, preferably high grade serous ovarian carcinoma, in the subject, and the treatment is selected from the group consisting of chemotherapy, surgery and post-surgery chemotherapy.

9. The method according to any one of claims 1 to 6, wherein the information is whether epithelial ovarian cancer, preferably high grade serous ovarian carcinoma, in the subject is of primary or secondary origin, and whether cancer of secondary origin is a secondary breast cancer metastatic cancer.

10. Use of a kit comprising a forward amplification primer having a sequence comprising SEQ ID NO: 1, a reverse amplification primer having a sequence comprising SEQ ID NO: 2 and a probe having a sequence comprising SEQ ID NO: 3 or specific oligonucleotides derived from one or more of the individual probe sets selected from the group comprising 1881_g_at, 1882_g_at, 208434_at; 221884_at and 226420_at, their fragments, derivatives, mutants and complementary sequences thereof in a method for obtaining information in relation to a medical condition in a subject according to any one of claims 1 to 9.

11. The use of a kit according to claim 10, wherein the copy number of said at least one gene is determined using at least one method selected from the group consisting of quantitative PCR assay, in situ hybridization, Southern blotting, multiplex ligation-dependent probe amplification (MLPA) and Quantitative Multiplex PCR of Short Fluorescent Fragments (QMPSF).

12. A set of oligonucleotides consisting of
a forward amplification primer having a nucleic acid sequence comprising SEQ ID NO: 1, a reverse amplification primer having a nucleic acid sequence comprising SEQ ID NO: 2 and a probe having a nucleic acid sequence comprising SEQ ID NO: 3,
wherein the set is capable of simultaneous detection and discrimination of complete protein-coding specific isoforms of EVI1 isoform only, MDS1 only and the combination of EVI1 and EVI1/MDS1 isoforms as defined by SEQ ID NO: 4, SEQ ID NO: 5, SEQ ID NO: 11, SEQ ID NO: 12, SEQ ID NO: 13, SEQ ID NO: 14 and SEQ ID NO: 15, and complete determination of transcription status of whole MECOM locus in an individual patient sample.

13. Use of specific oligonucleotides, derived from one or more of the individual probe sets selected from the group consisting of 1881_g_at, 1882_g_at, 208434_at; 221884_at and 226420_at, their fragments, derivatives, mutants and complementary sequences thereof, in a method for obtaining information in relation to a medical condition in a subject according to any one of claims 1 to 9.

**Patentansprüche**

1. *In-vitro*-Verfahren zum Erhalten von Informationen bezüglich eines medizinischen Leidens bei einem Individuum, wobei das Verfahren umfasst, dass in einer Probe des Individuums Folgendes bestimmt wird:

(i) Genexpressionsniveau von zumindest einem Gen im MECOM-Lokus, ausgewählt aus *EVI1* und *MDS1*; und/oder
(ii) Kopienzahl von zumindest einem Gen im MECOM-Lokus, ausgewählt aus *EVI1* und *MDS1*;

wobei die Informationen aus der Gruppe ausgewählt sind, die besteht aus:

a) dem, ob das Individuum epithelialen Eierstockkrebs, bevorzugt hochgradiges seröses Eierstockkarzinom, und/oder eine Prädisposition für epithelialen Eierstockkrebs, bevorzugt hochgradiges seröses Eierstockkarzinom, aufweist, wobei das Genexpressionsniveau gegenüber zumindest einem statistisch optimierten Genexpression-Cutoff-Wert, abgeleitet von mehreren Trainingsindividuen mit bekannter Diagnose, und/oder die zumindest eine Genkopienzahl gegenüber zumindest einem statistisch optimierten Genkopienzahl-Cutoff-Wert, abgeleitet von mehreren Trainingsindividuen mit bekannter Diagnose, ein Indikator/Indikatoren für die Informationen ist/sind, wobei der optimierte Cutoff-Wert mit den folgenden Schritten erhalten wird;

(i) Extrahieren des Expressionsniveaus oder des Kopienzahlniveaus eines Gens für jedes Trainingsindividuum;
(ii) Erhalten der kumulativen Verteilungsfunktion der Genexpressions- oder Kopienzahlniveaus zweier Mengen von Trainingsindividuen, wobei eine Menge mit einer positiven Diagnose assoziiert ist und eine Menge mit einer negativen Diagnose assoziiert ist; und
(iii) Auswählen eines Cutoff-Werts, der die Trainingsindividuen in zwei Gruppen teilt, basierend darauf, ob das Genexpressionsniveau oder das Kopienzahlniveau jedes Trainingsindividuums höher oder niedriger als der Cutoff-Wert ist, und der die Rate falsch positiver Ergebnisse und/oder falsch negativer Ergebnisse in der Diagnose mittels eindimensionaler statistischer Optimierung minimiert;

oder, falls die Expressions- und die Kopienzahlwerte in der gleichen Probe identifiziert werden, die optimalen Cutoffs für sowohl Expression als auch Kopienzahl gleichzeitig erhalten werden, indem die Raten falsch positiver Ergebnisse und/oder falsch negativer Ergebnisse mittels zweidimensionaler statistischer Optimierung minimiert werden;

wobei die Bestimmung epithelialen Eierstockkrebses Verwenden von PCR-Primern, umfassend einen Forward-Primer SEQ ID NO: 1 und einen Reverse-Primer SEQ ID NO: 2 und Sonde SEQ ID NO: 3, oder Verwenden zumindest eines der Sonden-Sets, ausgewählt aus 1881_g_at, 1882_g_at, 208434_at, 221884_at und 226420_at, umfasst;

b) Überlebensprognose für das Individuum, wenn das Individuum epithelialen Eierstockkrebs, bevorzugt hochgradiges seröses Eierstockkarzinom, aufweist, wobei das Expressionsniveau gegenüber zumindest einem statistisch optimierten Genexpression-Cutoff-Wert und/oder die zumindest eine Genkopienzahl gegenüber zumindest einem statistisch optimierten Genkopienzahl-Cutoff-Wert ein Indikator/Indikatoren für die Informationen ist/sind, wobei, zwecks primärer Patientenschichtung, die optimalen Kopienzahl- und Expression-Cutoff-Werte für jedes Gen mittels eines Zweischritt-Optimierungsalgorithmus wie folgt erhalten werden;

(i) Extrahieren des Expressionsniveaus und der Kopienzahl des Gens für jedes Trainingsindividuum;
(ii) statistisches Schätzen eines Kopienzahl-Cutoff-Werts und Einteilen der Trainingsindividuen in zwei Kohorten dementsprechend, ob die Kopienzahl des Gens über oder unter dem geschätzten Kopienzahl-Cutoff-Wert liegt; und
(iii) für jede Kohorte, Auswählen eines Genexpression-Cutoff-Werts, der die Trainingsindividuen in jeder Kohorte in zwei Gruppen teilt, basierend darauf, ob das Genexpressionsniveau jedes Trainingsindividuums höher oder niedriger als der Expression-Cutoff-Wert ist, wobei der Cutoff-Wert den Wald-Test-p-Wert von Kaplan-Meier-Überlebensmodellen minimiert;

wobei die Überlebensvorhersagebestimmung Verwenden von PCR-Primern, umfassend einen Forward-Primer SEQ ID NO: 1 und einen Reverse-Primer SEQ ID NO: 2 und Sonde SEQ ID NO: 3, oder Verwenden zumindest eines der Sonden-Sets, ausgewählt aus 1881_g_at, 1882_g_at, 221884_at und 226420_at, umfasst;

c) Wirksamkeit der Behandlung von epithelialem Eierstockkrebs, bevorzugt hochgradigem serösem Eierstockkarzinom, beim Individuum, wobei das Genexpressionsniveau gegenüber zumindest einem statistisch optimierten Genexpression-Cutoff-Wert und/oder die zumindest eine Genkopienzahl gegenüber zumindest einem optimierten Genkopienzahl-Cutoff-Wert ein Indikator/Indikatoren für die Informationen ist/sind;

wobei die Bestimmung der Wirksamkeit der Behandlung von epithelialem Eierstockkrebs Verwenden von PCR-Primern, umfassend einen Forward-Primer SEQ ID NO: 1 und einen Reverse-Primer SEQ ID NO: 2 und Sonde SEQ ID NO: 3, oder Verwenden zumindest eines der Sonden-Sets, ausgewählt aus 1881_g_at, 1882_g_at, 221884_at und 226420_at, umfasst;

d) dem, ob epithelialer Eierstockkrebs, bevorzugt hochgradiges seröses Eierstockkarzinom, beim Individuum primären oder sekundären Ursprungs ist, wobei das Genexpressionsniveau gegenüber zumindest einem statistisch optimierten Genexpression-Cutoff-Wert und/oder die zumindest eine Genkopienzahl gegenüber zumindest einem statistisch optimierten Genkopienzahl-Cutoff-Wert ein Indikator/Indikatoren für die Informationen ist/sind;

wobei die Bestimmung dessen, ob der epitheliale Eierstockkrebs primären oder sekundären Ursprungs ist, Verwenden von PCR-Primern, umfassend einen Forward-Primer SEQ ID NO: 1 und einen Reverse-Primer SEQ ID NO: 2 und Sonde SEQ ID NO: 3, oder Verwenden zumindest eines der Sonden-Sets, ausgewählt aus 226420_at, 221884_at und 208434_at, umfasst; und

e) dem, ob das Individuum hochgradiges seröses Eierstockkarzinom, Zervixkrebs, Eierstockendometriose, klarzelliges Nierenkarzinom und/oder eine Prädisposition für Zervixkrebs, Eierstockendometriose, klarzelliges Nierenkarzinom aufweist, wobei das Niveau gegenüber zumindest einem statistisch optimierten Genexpression-Cutoff-Wert und/oder die zumindest eine Genkopienzahl gegenüber zumindest einem statistisch optimierten Genkopienzahl-Cutoff-Wert ein Indikator/Indikatoren dafür ist/sind, dass das Individuum hochgradiges seröses Eierstockkarzinom, Zervixkrebs, Endometriose, klarzelliges Nierenkarzinom und/oder eine Prädisposition für Zervixkrebs, Endometriose oder klarzelliges Nierenkarzinom aufweist;

wobei die Bestimmung der Krebsart Verwenden von PCR-Primern, umfassend einen Forward-Primer SEQ ID NO: 1 und einen Reverse-Primer SEQ ID NO: 2 und Sonde SEQ ID NO: 3, oder Verwenden zumindest eines der Sonden-Sets, ausgewählt aus 226420_at, 1881_g_at, 1882_g_at, 221884_at und 208434_at, umfasst,

wobei das Genexpressionsniveau in a) - e) bestimmt wird aus zumindest einer Transkriptvariante oder Isoform, umfassend eine Nukleinsäuresequenz, ausgewählt aus der Gruppe, bestehend aus SEQ ID NO: 4, SEQ ID NO: 5, SEQ ID NO: 11, SEQ ID NO: 12, SEQ ID NO: 13, SEQ ID NO: 14 und SEQ ID NO: 15.

**2.** Verfahren nach Anspruch 1, wobei sich die Informationen auf epithelialen Eierstockkrebs, bevorzugt hochgradiges seröses Eierstockkarzinom, beziehen, der ein Adenokarzinom oder ein maligner Eierstockkrebs ist; und/oder wobei sich die Informationen auf epithelialen Eierstockkrebs, bevorzugt hochgradiges seröses Eierstockkarzinom, beziehen, der primärer epithelialer Eierstockkrebs ist.

**3.** Verfahren nach einem der vorstehenden Ansprüche, wobei:

das Genexpressionsniveau durch Messen der mRNA- oder Protein-Expression des Gens aus dem MECOM-Lokus bestimmt wird; und/oder
das Verfahren Bestimmen des Genexpressionsniveaus von zumindest einem weiteren Gen, ausgewählt aus der Gruppe, bestehend aus MUC16, WFDC2, ERBB1, ERBB2, ERBB3, EGF, NGR1 und TGFA, in der Probe umfasst.

**4.** Verfahren nach einem der vorstehenden Ansprüche, wobei das Trainingsstadium die folgenden Schritte für jedes Gen im MECOM-Lokus umfasst:

(i) für jedes von mehreren Trainingsindividuen mit bekannten Diagnosen bezüglich Eierstockkrebs, bevorzugt hochgradigem serösem Eierstockkarzinom, Bestimmen von Genexpressionsniveau und Kopienzahl des Gens beim Trainingsindividuum;
(ii) Schätzen einer Probenkopienzahl und Einteilen der Trainingsindividuen in zwei Kohorten dementsprechend, ob die Kopienzahl des Gens bei jedem Trainingsindividuum über oder unter der Probenkopienzahl liegt;
(iii) für jede Kohorte, Bestimmen eines Probenexpressionswerts, der die Trainingsindividuen in der Kohorte dementsprechend in zwei Gruppen teilt, ob das Genexpressionsniveau des Gens bei jedem Trainingsindividuum den Probenexpressionswert überschreitet, wobei der Probenexpressionswert ein maximales Maß des Unterschieds zwischen den zwei Gruppen erzielt;
(iv) Wiederholen der Schritte (ii) - (iii) durch Variieren der Probenkopienzahl in einem Bereich von Kopienzahlen, identifiziert bei den Trainingsindividuen, und Erhalten einer Kopienzahlverteilungskurve für das Gen; und
(v) Auswählen eines Kopienzahl-Cutoff-Werts als die Kopienzahl, assoziiert mit dem größten Maß des Unterschieds zwischen den zwei Gruppen einer Kohorte, und Auswählen von Expression-Cutoff-Werten als den Expressionswerten, bestimmt für die Kohorten, erhalten mit dem Kopienzahl-Cutoff-Wert,

wobei das Maß des Unterschieds zwischen den zwei Gruppen bevorzugt ein Maß des Unterschieds zwischen Überlebenskurven der zwei Gruppen umfasst; wobei die Diagnose für das Individuum, das Bestimmen der Überlebensprognose und/oder das Bestimmen der Wirksamkeit der Behandlung gegebenenfalls weiterhin die folgenden Schritte für jedes Gen im MECOM-Lokus umfassen:

Vergleichen der Kopienzahl des Gens des Individuums mit dem Kopienzahl-Cutoff-Wert für das Gen; wobei
falls die Kopienzahl des Gens des Individuums den Kopienzahl-Cutoff-Wert für das Gen überschreitet, weiterhin das Genexpressionsniveau des Gens des Individuums mit dem Expression-Cutoff-Wert, bestimmt für die Kohorte mit Kopienzahlen des Gens über dem Kopienzahl-Cutoff-Wert, verglichen wird;
falls die Kopienzahl des Gens des Individuums unter dem Kopienzahl-Cutoff-Wert für das Gen liegt, das Genexpressionsniveau des Gens des Individuums mit dem Expression-Cutoff-Wert, bestimmt für die Kohorte mit Kopienzahlen des Gens unter dem Kopienzahl-Cutoff-Wert, verglichen wird; und
Erhalten der Diagnose für das Individuum, Bestimmen der Überlebensprognose und/oder Bestimmen der Wirksamkeit der Behandlung auf Grundlage des Vergleichs zwischen dem Genexpressionsniveau des Gens des Individuums und dem jeweiligen Expression-Cutoff-Wert.

**5.** Verfahren nach Anspruch 4, zum Bestimmen der Überlebensprognose für das Individuum, wobei das Verfahren weiterhin die Schritte umfasst:

(i) Parametrisierung einer Abhängigkeit zwischen einer Patientenkohortenfraktion und der Kopienzahl des Gens in der Menge von Trainingsindividuen;
(ii) Parametrisierung einer Abhängigkeit zwischen der Patientenkohortenfraktion und der Überlebenszeit in der Menge von Trainingsindividuen, wobei die Überlebenszeit der Trainingsindividuen bevorzugt auf einer letzten Follow-up-Zeit für die Trainingsindividuen beruht; und

(iii) Verwenden der Kopienzahl des Individuums, um die Patientenkohortenfraktion aus der Abhängigkeit von (i) zu bestimmen, und Verwenden der Patientenkohortenfraktion des Individuums, um eine geschätzte Überlebenszeit des Individuums aus Abhängigkeit (ii) zu bestimmen.

6. Verfahren nach Anspruch 4, zum Bestimmen der Behandlungswirksamkeit, weiterhin umfassend die Schritte:

(i) Parametrisieren einer Abhängigkeit zwischen der Kopienzahl des Gens des Trainingsindividuums und einem möglichen Behandlungsansprechen des Trainingsindividuums, ausgewählt aus einer Gruppe, bestehend aus:

- einem vollständigen Ansprechen;
- einem unvollständigen Ansprechen; und
- keinem Ansprechen; und

(ii) Verwenden der Kopienzahl des Individuums, um ein geschätztes mögliches Behandlungsansprechen des Individuums zu bestimmen.

7. Verfahren nach einem der vorstehenden Ansprüche, wobei sich die Informationen auf den epithelialen Eierstockkrebs, bevorzugt das hochgradige seröse Eierstockkarzinom, beziehen, und das Verfahren in der Lage ist zum: Bestimmen, ob der Eierstockkrebs beim Individuum primärer oder sekundärer Eierstockkrebs ist; oder ob der Eierstockkrebs ein Malignitätspotenzial beim Individuum aufweist.

8. Verfahren nach einem der Ansprüche 1 bis 6, wobei die Informationen in der Wirksamkeit der Behandlung von epithelialem Eierstockkrebs, bevorzugt hochgradigem serösem Eierstockkarzinom, beim Individuum bestehen, und die Behandlung aus der Gruppe ausgewählt ist, die aus Chemotherapie, Operation und postoperativer Chemotherapie besteht.

9. Verfahren nach einem der Ansprüche 1 bis 6, wobei die Informationen darin bestehen, ob epithelialer Eierstockkrebs, bevorzugt hochgradiges seröses Eierstockkarzinom, beim Individuum primären oder sekundären Ursprungs ist, und ob Krebs sekundären Ursprungs ein sekundärer, metastatischer Brustkrebs ist.

10. Verwendung eines Kits, umfassend einen Forward-Amplifikations-Primer mit einer SEQ ID NO: 1 umfassenden Sequenz, einen Reverse-Amplifikations-Primer mit einer SEQ ID NO: 2 umfassenden Sequenz und eine Sonde mit einer SEQ ID NO: 3 umfassenden Sequenz oder spezifische Oligonukleotide, stammend von einem oder mehreren der Individual-Sonden-Sets, ausgewählt aus der Gruppe, umfassend 1881_g_at, 1882_g_at, 208434_at; 221884_at und 226420_at, ihre Fragmente, Derivate, Mutanten und komplementäre Sequenzen davon, in einem Verfahren zum Erhalten von Informationen bezüglich eines medizinischen Leidens bei einem Individuum nach einem der Ansprüche 1 bis 9.

11. Verwendung eines Kits nach Anspruch 10, wobei die Kopienzahl des zumindest einen Gens mittels zumindest eines Verfahrens bestimmt wird, das aus der Gruppe ausgewählt ist, die aus quantitativem PCR-Assay, *In-situ*-Hybridisierung, Southern Blot, multiplexer ligationsabhängiger Sondenamplifikation (MLPA) und quantitativer multiplexer PCR kurzer fluoreszierender Fragmente (QMPSF) besteht.

12. Set von Oligonukleotiden, bestehend aus
einem Forward-Amplifikations-Primer mit einer SEQ ID NO: 1 umfassenden Nukleinsäuresequenz, einem Reverse-Amplifikations-Primer mit einer SEQ ID NO: 2 umfassenden Nukleinsäuresequenz und einer Sonde mit einer SEQ ID NO: 3 umfassenden Nukleinsäuresequenz,
wobei das Set in der Lage ist zur gleichzeitigen Detektion und Diskriminierung vollständiger proteincodierender spezifischer Isoformen von nur EVI1-Isoform, nur MDS1 und der Kombination von EVI1- und EVI1/MDSI-Isoformen, wie definiert durch SEQ ID NO: 4, SEQ ID NO: 5, SEQ ID NO: 11, SEQ ID NO: 12, SEQ ID NO: 13, SEQ ID NO: 14 und SEQ ID NO: 15, und zur vollständigen Bestimmung des Transkriptionsstatus des gesamten MECOM-Lokus in einer individuellen Probe eines Patienten.

13. Verwendung von spezifischen Oligonukleotiden, stammend von einem oder mehreren der individuellen Sonden-Sets, ausgewählt aus der Gruppe, bestehend aus 1881_g_at, 1882_g_at, 208434_at; 221884_at und 226420_at, ihren Fragmenten, Derivaten, Mutanten und komplementären Sequenzen davon, in einem Verfahren zum Erhalten von Informationen bezüglich eines medizinischen Leidens bei einem Individuum nach einem der Ansprüche 1 bis 9.

**Revendications**

1. Méthode *in vitro* pour l'obtention d'une information relativement à un état médical chez un sujet, la méthode comprenant la détermination dans un échantillon du sujet:

   (i) du niveau d'expression de gène d'au moins un gène dans le locus MECOM, choisi parmi *EVI1* et *MDS1*; et/ou
   (ii) du nombre de copies d'au moins un gène dans le locus MECOM choisi parmi *EVI1* et *MDS1*;

   dans laquelle l'information est choisie dans le groupe constitué de:

   a) si le sujet présente un cancer ovarien épithélial, de préférence un carcinome ovarien séreux de grade élevé et/ou une prédisposition à un cancer ovarien épithélial, de préférence un carcinome ovarien séreux de grade élevé, où le niveau d'expression de gène vis-à-vis d'au moins une valeur de seuil d'expression de gène statistiquement optimisée dérivée d'une pluralité de sujets d'entraînement avec un diagnostic connu et/ou le nombre de copies d'au moins un gène vis-à-vis d'au moins une valeur de seuil de nombre de copies de gène statistiquement optimisée dérivée d'une pluralité de sujets d'entraînement avec un diagnostic connu est/sont une indication de ladite information, dans laquelle ladite valeur de seuil optimisée est obtenue avec les étapes suivantes:

   (i) extraction du niveau d'expression ou du niveau de nombre de copies d'un gène pour chaque sujet d'entraînement;
   (ii) obtention de la fonction de distribution cumulée des niveaux d'expression de gène ou de nombre de copies de deux groupes de sujets d'entraînement, un groupe étant associé à un diagnostic positif et un groupe étant associé à un diagnostic négatif; et
   (iii) sélection d'une valeur de seuil qui divise les sujets d'entraînement en deux groupes, basé sur si le niveau d'expression de gène ou le niveau de nombre de copies de chaque sujet d'entraînement est supérieur ou inférieur à la valeur de seuil, et qui minimise le taux de faux positif et/ou de faux négatif dans le diagnostic en utilisant une optimisation statistique à une dimension;

   ou, si les valeurs d'expression et de nombre de copies sont identifiées dans le même échantillon, les seuils optimaux pour à la fois l'expression et le nombre de copies sont obtenus simultanément en minimisant les taux de faux positif et/ou de faux négatif en utilisant une optimisation statistique à deux dimensions; dans laquelle ladite détermination de cancer ovarien épithélial comprend l'utilisation d'amorces PCR comprenant une amorce sens SEQ ID NO: 1 et une amorce anti-sens SEQ ID NO: 2 et une sonde SEQ ID NO: 3 ou l'utilisation d'au moins un des groupes de sondes choisis parmi 1881_g_at, 1882_g_at, 208434_at, 221884_at et 226420_at;
   b) pronostic de survie du sujet lorsque le sujet présente un cancer ovarien épithélial, de préférence un carcinome ovarien séreux de grade élevé, où le niveau d'expression vis-à-vis d'au moins une valeur de seuil d'expression de gène statistiquement optimisée et/ou le nombre de copies d'au moins un gène vis-à-vis d'au moins une valeur de seuil de nombre de copies de gène statistiquement optimisée est/sont une indication de ladite information, où, pour une stratification de patients principale, les valeurs de seuil de nombre de copies et d'expression optimales sont obtenues pour chaque gène en utilisant un algorithme d'optimisation à deux étapes comme suit:

   (i) extraction du niveau d'expression ou du nombre de copies du gène pour chaque sujet d'entraînement;
   (ii) estimation statistique d'une valeur de seuil de nombre de copies et division des sujets d'entraînement en deux cohortes selon si le nombre de copies du gène est supérieur ou inférieur à la valeur de seuil de nombre de copies estimée; et
   (iii) pour chacune desdites cohortes, sélection d'une valeur de seuil d'expression de gène qui divise les sujets d'entraînement dans chaque cohorte en deux groupes, basé sur si le niveau d'expression de gène de chaque sujet d'entraînement est supérieur ou inférieur à la valeur de seuil d'expression, où la valeur de seuil minimise la valeur-p du test de Wald des modèles de survie de Kaplan-Meier;

   dans laquelle ladite détermination de prédiction de survie comprend l'utilisation d'amorces PCR comprenant une amorce sens SEQ ID NO: 1 et une amorce anti-sens SEQ ID NO: 2 et une sonde SEQ ID NO: 3 ou l'utilisation d'au moins un des groupes de sondes choisis parmi 1881_g_at, 1882_g_at, 221884_at et 226420_at;
   c) efficacité de traitement d'un cancer ovarien épithélial, de préférence un carcinome ovarien séreux de grade élevé, chez le sujet, où le niveau d'expression de gène vis-à-vis d'au moins une valeur de seuil d'expression de gène statistiquement optimisée et/ou le nombre de copies d'au moins un gène vis-à-vis d'au moins une valeur de seuil de nombre de copies de gène optimisée sont une indication de ladite information;

dans laquelle ladite détermination d'efficacité de traitement d'un cancer ovarien épithélial comprend l'utilisation d'amorces PCR comprenant une amorce sens SEQ ID NO: 1 et une amorce anti-sens SEQ ID NO: 2 et une sonde SEQ ID NO: 3 ou l'utilisation d'au moins un des groupes de sondes choisis parmi 1881_g_at, 1882_g_at, 221884_at et 226420_at;

d) si un cancer ovarien épithélial, de préférence un carcinome ovarien séreux de grade élevé, chez le sujet est d'origine principale ou secondaire, où le niveau d'expression de gène vis-à-vis d'au moins une valeur de seuil d'expression de gène statistiquement optimisée et/ou le nombre de copies d'au moins un gène vis-à-vis d'au moins une valeur de seuil de nombre de copies de gène statistiquement optimisée sont une indication de ladite information;

dans laquelle ladite détermination si le cancer ovarien épithélial est d'origine principale ou secondaire comprend l'utilisation d'amorces PCR comprenant une amorce sens SEQ ID NO: 1 et une amorce anti-sens SEQ ID NO: 2 et une sonde SEQ ID NO: 3 ou l'utilisation d'au moins un des groupes de sondes choisis parmi 226420_at, 221884_at et 208434_at; et

e) si le sujet présente un carcinome ovarien séreux de grade élevé, un cancer du col de l'utérus, une endométriose ovarienne, un carcinome rénal à cellules claires et/ou une prédisposition à un cancer du col de l'utérus, une endométriose ovarienne, un carcinome rénal à cellules claires, où le niveau vis-à-vis d'au moins une valeur de seuil d'expression de gène statistiquement optimisée et/ou le nombre de copies d'au moins un gène vis-à-vis d'au moins une valeur de seuil de nombre de copies de gène statistiquement optimisée sont une indication du sujet qui présente un carcinome ovarien séreux de grade élevé, un cancer du col de l'utérus, une endométriose, un carcinome rénal à cellules claires et/ou une prédisposition à un cancer du col de l'utérus, une endométriose, un carcinome rénal à cellules claires;

dans laquelle ladite détermination du type de cancer comprend l'utilisation d'amorces PCR comprenant une amorce sens SEQ ID NO: 1 et une amorce anti-sens SEQ ID NO: 2 et une sonde SEQ ID NO: 3 ou l'utilisation d'au moins un des groupes de sondes choisis parmi 226420_at, 1881_g_at, 1882_g_at, 221884_at et 208434_at,

dans laquelle ledit niveau d'expression de gène dans a)-e) est déterminé à partir d'au moins un variant ou une isoforme de transcrit comprenant une séquence d'acides nucléiques choisie dans le groupe constitué de la SEQ ID NO: 4, la SEQ ID NO: 5, la SEQ ID NO: 11, la SEQ ID NO: 12, la SEQ ID NO: 13, la SEQ ID NO: 14 et la SEQ ID NO: 15.

2. Méthode selon la revendication 1, dans laquelle l'information est relativement à un cancer ovarien épithélial, de préférence un carcinome ovarien séreux de grade élevé, qui est un adénocarcinome ou un cancer ovarien malin; et/ou dans laquelle l'information est relativement à un cancer ovarien épithélial, de préférence un cancer ovarien séreux de grade élevé, qui est un cancer ovarien épithélial principal.

3. Méthode selon l'une quelconque des revendications précédentes, dans laquelle:

le niveau d'expression de gène est déterminé en mesurant l'expression d'ARNm ou de protéine du gène à partir du locus MECOM; et/ou

la méthode comprend la détermination du niveau d'expression de gène d'au moins un gène supplémentaire choisi dans le groupe constitué de MUC16, WFDC2, ERBB1, ERBB2, ERBB3, EGF, NGR1 et TGFA, dans l'échantillon.

4. Méthode selon l'une quelconque des revendications précédentes, dans laquelle la phase d'entraînement comprend les étapes suivantes pour chacun desdits gènes dans le locus MECOM:

(i) pour chacun d'une pluralité de sujets d'entraînement avec des diagnostics connus en rapport avec un cancer ovarien, de préférence un carcinome ovarien séreux de grade élevé, la détermination du niveau d'expression de gène et du nombre de copies du gène chez le sujet d'entraînement;

(ii) l'estimation d'un nombre de copies d'échantillon et la division des sujets d'entraînement en deux cohortes selon si le nombre de copies du gène chez chaque sujet d'entraînement est supérieur ou inférieur au nombre de copies d'échantillon;

(iii) pour chacune desdites cohortes, la détermination d'une valeur d'expression d'échantillon qui divise les sujets d'entraînement dans la cohorte en deux groupes selon si le niveau d'expression de gène du gène chez chaque sujet d'entraînement dépasse la valeur d'expression d'échantillon, où la valeur d'expression d'échantillon atteint une mesure maximum de différence entre les deux groupes;

(iv) la répétition des étapes (ii)-(iii) en variant le nombre de copies d'échantillon dans un intervalle de nombres de copies identifiés chez les sujets d'entraînement et l'obtention d'une courbe de distribution de nombres de copies pour le gène; et

(v) la sélection d'une valeur de seuil de nombre de copies comme le nombre de copies associé à la mesure la plus grande de différence entre les deux groupes d'une cohorte et la sélection des valeurs de seuil d'expression comme les valeurs d'expression déterminées pour les cohortes obtenues avec la valeur de seuil de nombre de copies,

la mesure de différence entre les deux groupes comprenant de préférence une mesure de différence entre les courbes de survie des deux groupes; où le diagnostic du sujet, en déterminant le pronostic de survie et/ou en déterminant l'efficacité de traitement comprend en outre optionnellement les étapes suivantes pour chacun desdits gènes dans le locus MECOM:

la comparaison du nombre de copies du gène du sujet vis-à-vis de la valeur de seuil de nombre de copies pour le gène;
dans laquelle:

si le nombre de copies du gène du sujet dépasse la valeur de seuil de nombre de copies pour le gène, la comparaison en outre du niveau d'expression de gène du gène du sujet vis-à-vis de la valeur de seuil d'expression déterminée pour la cohorte avec des nombres de copies du gène supérieurs à la valeur de seuil de nombre de copies;
si le nombre de copies du gène du sujet est inférieur à la valeur de seuil de nombre de copies pour le gène, la comparaison du niveau d'expression de gène du gène du sujet vis-à-vis de la valeur de seuil d'expression déterminée pour la cohorte avec des nombres de copies du gène inférieurs à la valeur de seuil de nombre de copies; et
l'obtention d'un diagnostic du sujet, en déterminant le pronostic de survie et/ou en déterminant l'efficacité de traitement basé sur la comparaison entre le niveau d'expression de gène du gène du sujet et la valeur de seuil d'expression respective.

5. Méthode selon la revendication 4, pour la détermination du pronostic de survie du sujet, où la méthode comprend en outre les étapes de:

(i) paramétrage d'une dépendance entre une fraction de cohorte de patients et le nombre de copies du gène dans le groupe de sujets d'entraînement;
(ii) paramétrage d'une dépendance entre la fraction de cohorte de patients et le temps de survie dans le groupe de sujets d'entraînement, le temps de survie des sujets d'entraînement étant de préférence basé sur un temps de dernier suivi pour les sujets d'entraînement; et
(iii) l'utilisation du nombre de copies du sujet pour déterminer la fraction de cohorte de patients à partir de la dépendance de (i) et l'utilisation de la fraction de cohorte de patients du sujet pour déterminer un temps de survie estimé du sujet à partir de la dépendance de (ii).

6. Méthode selon la revendication 4, pour la détermination de l'efficacité de traitement, comprenant en outre les étapes de:

(i) paramétrage d'une dépendance entre le nombre de copies du gène du sujet d'entraînement et une réponse au traitement possible du sujet d'entraînement choisie dans le groupe constitué de:

- une réponse complète;
- une réponse incomplète; et
- une réponse nulle; et

(ii) l'utilisation du nombre de copies du sujet pour déterminer une réponse au traitement possible estimée du sujet.

7. Méthode selon l'une quelconque des revendications précédentes, dans laquelle l'information est relativement à un cancer ovarien épithélial, de préférence un carcinome ovarien séreux de grade élevé et la méthode est capable de: déterminer si le cancer ovarien chez le sujet est un cancer ovarien principal ou secondaire; ou si le cancer ovarien a un potentiel malin chez le sujet.

8. Méthode selon l'une quelconque des revendications 1 à 6, dans laquelle l'information est l'efficacité de traitement d'un cancer ovarien épithélial, de préférence un carcinome ovarien séreux de grade élevé, chez le sujet, et le traitement est choisi dans le groupe constitué de: chimiothérapie, chirurgie et chimiothérapie après chirurgie.

9. Méthode selon l'une quelconque des revendications 1 à 6, dans laquelle l'information est si le cancer ovarien épithélial, de préférence un carcinome ovarien séreux de grade élevé, chez le sujet est d'origine principale ou secondaire et si le cancer d'origine secondaire est un cancer métastasique de cancer du sein secondaire.

10. Utilisation d'un kit comprenant une amorce d'amplification sens possédant une séquence comprenant la SEQ ID NO: 1, une amorce d'amplification anti-sens possédant une séquence comprenant la SEQ ID NO: 2 et une sonde possédant une séquence comprenant la SEQ ID NO: 3 ou des oligonucléotides spécifiques dérivés d'un ou de plusieurs des groupes de sondes individuelles choisis dans le groupe comprenant 1881_g_ at, 1882_g_at, 208434_at, 221884_at et 226420_at, leurs fragments, dérivés, des mutants et des séquences complémentaires de ceux-ci dans une méthode pour l'obtention d'une information relativement à un état médical chez un sujet selon l'une quelconque des revendications 1 à 9.

11. Utilisation du kit selon la revendication 10, dans laquelle le nombre de copies dudit au moins un gène est déterminé en utilisant au moins une méthode choisie dans le groupe constitué de: essai PCR quantitatif, hybridation in situ, transfert de type Southern, amplification par sonde dépendant d'une ligature multiplex (MLPA) et PCR multiplex quantitative de fragments fluorescents courts (QMPSF).

12. Groupe d'oligonucléotides constitué de:

une amorce d'amplification sens possédant une séquence d'acides nucléiques comprenant la SEQ ID NO: 1, une amorce d'amplification anti-sens possédant une séquence d'acides nucléiques comprenant la SEQ ID NO: 2 et une sonde possédant une séquence d'acides nucléiques comprenant la SEQ ID NO: 3, où le groupe est capable de détection et de discrimination simultanées d'isoformes spécifiques de codage de protéine complètes de l'isoforme de EVI1 seule, MDS1 seule et de la combinaison des isoformes de EVI1 et EVI1/MDS1 telles que définies par la SEQ ID NO: 4, la SEQ ID NO: 5, la SEQ ID NO: 11, la SEQ ID NO: 12, la SEQ ID NO: 13, la SEQ ID NO: 14 et la SEQ ID NO: 15 et de détermination complète de l'état de transcription du locus MECOM total dans un échantillon de patient individuel.

13. Utilisation d'oligonucléotides spécifiques, dérivés d'un ou de plusieurs des groupes de sondes individuelles choisis dans le groupe constitué de: 1881_g_at, 1882_g_at, 208434_at, 221884_at et 226420_at, leurs fragments, dérivés, des mutants et des séquences complémentaires de ceux-ci, dans une méthode pour l'obtention d'une information relativement à un état médical chez un sujet selon l'une quelconque des revendications 1 à 9.

**FIGURE 1**

**FIGURE 2**

A) Primary ovarian tumor markers

B) Malignancy potential markers

FIGURE 3

FIGURE 4

Identification of primary/secondary ovarian tumours    Identification of LMP/malignancy of ovarian tumours

**FIGURE 5**

## ERBB family receptors

## Signalling molecules - ligands of the ERBB family receptors

**FIGURE 6**

**FIGURE 7**

42

FIGURE 8

Long follow-up: death later than 300 days after surgery

Short follow-up: death earlier than 300 days after surgery

## FIGURE 9

Long follow-up: death later than 300 days after surgery

Short follow-up: death earlier than 300 days after surgery

## FIGURE 10

**FIGURE 11**

A) Dead without secondary chemotherapy
by or living after 600 days

B) Dead with secondary chemotherapy
by or living after 600 days

**FIGURE 12**

**FIGURE 13**

**FIGURE 14**

**A)**

y= x*a^ 2+ bx+ c

Patient cohort fraction vs Last follow-up time, days

**B)**

EVI1 P-value vs Last follow-up time, days

**C)**

MDS1 P-value vs Last follow-up time, days

Patient cohorts

| | | |
|---|---|---|
| ● ● | **Complete response, tau= 0.951** | |
| – – | a= -1.1e-07 b= 0.000459 c= -0.036 | |
| ● ● | **Incomplete response, tau= 0.467** | |
| ─── | a= -1.9e-07 b= 0.000522 c= 0.111 | |
| ● ● | **Null, tau= -0.962** | |
| ···· | a= 3e-07 b= -0.000981 c= 0.925 | |

# FIGURE 15

**FIGURE 16**

EVI1 CN values distribution

Patient cohorts

Patient X with EVI1 CN=4.2

**Primary therapy outcome prediction:**
Complete response P=0.58
No response: P=0.42
Estimated surivival 900-1200 days

**FIGURE 17**

A

Probe 1881_g_at expression

B

Probe 1882_g_at expression

C

OC patients by stage

**FIGURE 18**

A

Probe 1881_g_at expression

B

Probe 1882_g_at expression

**FIGURE 19**

50

**FIGURE 20**

**FIGURE 21**

FIGURE 22

FIGURE 23

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 101386888
- WO 0118542 A
- WO 02079411 A

### Non-patent literature cited in the description

- **SAMBROOK ; RUSSEL.** Molecular Cloning: A Laboratory Manual. Cold Springs Harbor Laboratory, 2001
- **ANGLESIO MS ; ARNOLD JM ; GEORGE J ; TINKER AV ; TOTHILL R et al.** Mutation of erbb2 provides a novel alternative mechanism for the ubiquitous activation of ras-mapk in ovarian serous low malignant potential tumors. *Mol Cancer Res,* 2008, vol. 6, 1678-90
- **BOWEN NJ ; WALKER LD ; MATYUNINA LV ; LOGANI S ; TOTTEN KA et al.** Gene expression profiling supports the hypothesis that human ovarian surface epithelia are multipotent and capable of serving as ovarian cancer initiating cells. *BMC Med Genomics,* 2009, vol. 2, 71
- **EDGAR R.** Gene expression omnibus: Ncbi gene expression and hybridization array data repository. *Nucleic Acids Research,* 2002, vol. 30, 207
- **JAZAERI AA ; FERRISS JS ; BRYANT JL ; DALTON MS ; DUTTA A.** Evaluation of evi1 and evi1s (delta324) as potential therapeutic targets in ovarian cancer. *Gynecol Oncol,* 2010, vol. 118, 189-95
- **KERR MK ; CHURCHILL GA.** Statistical design and the analysis of gene expression microarray data. *Genet Res,* 2001, vol. 77, 123-8
- **LI C ; HUNG WONG W.** Model-based analysis of oligonucleotide arrays: model validation, design issues and standard error application. *Genome Biology,* 2001, vol. 2 (8
- **MCLENDON RE ; FRIEDMAN AH ; D BD.** Comprehensive genomic characterization defines human glioblastoma genes and core pathways. *Nature,* 2008, vol. 455, 1061-8
- **MEYNIEL JP ; COTTU PH ; DECRAENE C ; STERN MH ; COUTURIER J et al.** A genomic and transcriptomic approach for a differential diagnosis between primary and secondary ovarian carcinomas in patients with a previous history of breast cancer. *BMC Cancer,* 2010, vol. 10, 222
- **SUNDE JS ; DONNINGER H ; WU K ; JOHNSON ME ; PESTELL RG et al.** Expression profiling identifies altered expression of genes that contribute to the inhibition of transforming growth factor-beta signaling in ovarian cancer. *Cancer Res,* 2006, vol. 66, 8404-12
- **NANJUNDAN M ; NAKAYAMA Y ; CHENG KW ; LAHAD J ; LIU J ; LU K ; KUO WL ; SMITH-MCCUNE K ; FISHMAN D. GRAY JW ; MILLS GB.** Amplification of MDS1/EVI1 and EVI1, Located in the 3q26.2 Amplicon, Is Associated with Favorable Patient Prognosis in Ovarian Cancer. *Cancer Res,* 2007, vol. 67, 3074-3084
- **MORENO CS ; MATYUNINA L ; DICKERSON EB ; SCHUBERT N ; BOWEN NJ ; LOGANI S ; BENIGNO BB ; MCDONALD JF.** Evidence that p53-mediated cell-cycle-arrest inhibits chemotherapeutic treatment of ovarian carcinomas. *PLoS One,* 2007, vol. 2, e441
- **SCOTTO L ; NARAYAN G ; NANDULA SV ; ARIAS-PULIDO H ; SUBRAMANIYAM S ; SCHNEIDER A ; KAUFMANN AM ; WRIGHT JD ; POTHURI B ; MANSUKHANI M.** Identification of copy number gain and overexpressed genes on chromosome arm 20q by an integrative genomic approach in cervical cancer: potential role in progression. *Genes Chromosomes Cancer,* 2008, vol. 47, 755-65
- **HEVER A ; ROTH RB ; HEVEZI P ; MARIN ME ; ACOSTA JA ; ACOSTA H ; ROJAS J ; HERRERA R ; GRIGORIADIS D ; WHITE E.** Human endometriosis is associated with plasma cells and overexpression of B lymphocyte stimulator. *Proc Natl Acad Sci U S A.,* 2007, vol. 104, 12451-6
- **GUMZ ML ; ZOU H ; KREINEST PA ; CHILDS AC ; BELMONTE LS ; LEGRAND SN ; WU KJ ; LUXON BA ; SINHA M ; PARKER AS.** Secreted frizzled-related protein 1 loss contributes to tumor phenotype of clear cell renal cell carcinoma. *Clin Cancer Res.,* 2007, vol. 13, 4740-9
- **LIVAK KJ ; SCHMITTGEN TD.** Analysis of relative gene expression data using real-time quantitative PCR and the 2(-Delta Delta C(T)) Method. *Methods.,* 2001, vol. 25, 402-8

- **FERRIS.** Poster Session. *Gynecologic Oncology,* 01 March 2008, vol. 108 (3
- **BROOKS D J et al.** Expression of the Zine Finger Gene EVI-1 in Ovarian and Other Cancers. *British J. of Cancer,* 1996, vol. 74 (10
- **JOHNSON et al.** Identification of EVI1 amplification in ovarian cancer by oligonucleotide array comparative genomic hybridization. *Can. Res.,* 2007
- Unique expression of the human Evi-1gene in an endometrial carcinoma cell line: sequences of cDNAs and structure of alternatively spliced transcripts. **MORISHITA K et al.** Database Medline. US National Library of Medicine (NLM)
- **NANJUNDAN M. et al.** Amplification of MDS1/EVI1 and EVI1 located in the 3q26.2 amplicon, is associated with favorable patient prognosis in ovarian cancer. *Can. Res.,* 2007, vol. 67